# EUROPÄISCHE PATENTSCHRIFT

(11) **EP 1 377 570 B1**
(45) Veröffentlichungstag und Bekanntmachung des Hinweises auf die Patenterteilung: **13.05.2009**
(21) Anmeldenummer: 02757721.2
(22) Anmeldetag: 25.03.2002
(51) Int. Cl.: C07D 405/04, C07D 213/85

(54) **SUBSTITUIERTE 2-CARBA-3,5-DICYANO-4-ARYL-6-AMINOPYRIDINE UND IHRE VERWENDUNG ALS ADENOSINREZEPTOR SELEKTIVE LIGANDEN**
SUBSTITUTED 2-CARBA-3,5-DICYANO-4-ARYL-6-AMINOPYRIDINES AND THE USE OF THE SAME AS SELECTIVE LIGANDS OF THE ADENOSINE RECEPTOR
2-CARBA-3,5-DICYANO-4-ARYL-6-AMINOPYRIDINES SUBSTITUES ET LEUR UTILISATION EN TANT QUE LIGANDS SELECTIFS DU RECEPTEUR DE L'ADENOSINE

(30) Priorität: 30.03.2001 DE 10115945
(43) Veröffentlichungstag der Anmeldung: 07.01.2004
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); VAUPEL, Andrea, CH-4125 Riehen (CH); HÜBSCH, Walter, 42113 Wuppertal (DE); DIEDRICHS, Nicole, 42103 Wuppertal (DE); KRAHN, Thomas, 58135 Hagen (DE); DEMBOWSKY, Klaus, Boston, MA 02118 (US); STASCH, Johannes-Peter, 42651 Solingen (DE); SHIMADA, Mitsuyuki, Nara 630-8323 (JP)
(86) Internationale Anmeldenummer: PCT/EP2002/003303
(87) Internationale Veröffentlichungsnummer: WO 2002/079196

(56) Entgegenhaltungen:
- EP-A- 0 282 904
- EP-A- 0 705 820
- WO-A-01/25210
- WO-A-01/62233
- WO-A-97/27177
- WO-A-99/19302

## Beschreibung

Die vorliegende Erfindung betrifft substituierte 2-Carba-3,5-dicyano-4-aryl-6-aminopyridine, ein Verfahren zu ihrer Herstellung und ihre Verwendung als Arzneimittel.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehirn).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosinrezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M.E. Olah, H. Ren, J. Ostrowski, K.A. Jacobson, G.L. Stiles, "Cloning, expression, and characterization of the unique bovine A1 adenosine receptor. Studies on the ligand binding site by site-directed mutagenesis." in J. Biol. Chem. 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K.N. Klotz, J. Hessling, J. Hegler, C. Owman, B. Kull, B.B. Fredholm, M.J. Lohse, "Comparative pharmacology of human adenosine receptor subtypes - characterization of stably transfected receptors in CHO cells" in Naunyn Schmiedebergs Arch. Pharmacol. 357 (1998) Seiten 1-9, deren Offenbarung hiermit im vollen Umfang durch Bezugnahme eingeschlossen ist).

Bei den aus dem Stand der Technik bekannten, als "adenosinrezeptor-spezifisch" geltenden Liganden handelt es sich überwiegend um Derivate auf Basis des natürlichen Adenosins (S.-A. Poulsen und R.J. Quinn, "Adenosine receptors: new opportunities for future drugs" in Bioorganic and Medicinal Chemistry 6 (1998) Seiten 619-641; K. J. Broadley, "Drugs modulating adenosine receptors as potential therapeutic agents for cardiovascular diseases" in Exp. Opin. Ther. Patents 10 (2000) Seiten 1669-1692). Die aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Daher werden sie aufgrund der zuvor genannten Nachteile überwiegend nur für experimentelle Zwecke verwendet.

Aufgabe der vorliegenden Erfindung ist es, pharmakologisch aktive Substanzen aufzufinden oder bereitzustellen, die für die Prophylaxe und/oder Behandlung verschiedenster Erkrankungen, insbesondere Erkrankungen des Herz-Kreislauf-Systems (kardiovaskuläre Erkrankungen), geeignet sind und dabei vorzugsweise als Adenosinrezeptor-selektive Liganden wirken.

Die vorliegende Erfindung betrifft Verbindungen der Formel (I) 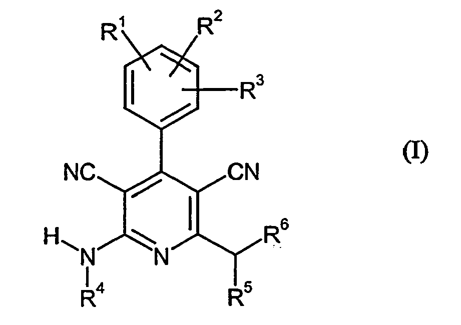 worin
R¹, R² und R³ unabhängig voneinander (C₁-C₈)-Alkyl, das bis zu dreifach durch Hydroxy, (C₁-C₄)-Alkoxy, (C₃-C₇)-Cycloalkyl, (C₂-C₄)-Alkenyl, (C₂-C₄)-Alkinyl, Halogen oder (C₆-C₁₀)-Aryloxy substituiert sein kann, (C₆-C₁₀)-Aryl, das bis zu dreifach durch Halogen, Nitro, (C₁-C₄)-Alkoxy, Carboxyl, (C₁-C₄)-Alkoxycarbonyl oder Mono- oder Di-(C₁-C₄)-alkylamino substituiert sein kann,
(C₁-C₈)-Alkoxy, das durch Hydroxy, (C₁-C₄)-Alkoxy, (C₃-C₇)-Cycloalkyl, (C₂-C₄)-Alkenyl, (C₆-C₁₀)-Aryl, 5- oder 6-gliedriges Heteroaryl mit bis zu drei Heteroatomen aus der Reihe N, O und/oder S, (C₆-C₁₀)-Aryloxy, Halogen, Cyano, (C₁-C₄)-Alkoxycarbonyl, (C₁-C₄)-Alkanoyloxy, Amino oder Mono- oder Di-(C₁-C₄)-alkylamino substituiert sein kann,
Wasserstoff, Hydroxy, Halogen, Nitro, Cyano oder -NH-C(O)- R⁷ bedeuten,
worin
R⁷ (C₁-C₈)-Alkyl, das durch Hydroxy oder (C₁-C₄)-Alkoxy substituiert sein kann, (C₃-C₇)-Cycloalkyl oder (C₆-C₁₀)-Aryl, das bis zu dreifach durch, unabhängig voneinander, durch Halogen, Nitro, (C₁-C₄)-Alkoxy, Carboxyl, (C₁-C₄)-Alkoxycarbonyl oder Mono- oder Di-(C₁-C₄)-alkylamino substituiert sein kann, bedeutet,
oder
R¹ und R² an benachbarte Phenylringatome gebunden sind und mit den beiden Ringkohlenstoffatomen gemeinsam einen 5- bis 7-gliedrigen gesättigten oder partiell ungesättigten Heterocyclus mit einem oder zwei Heteroatomen aus der Reihe N, O und/oder S bilden, der durch (C₁-C₄)-Alkyl oder Oxo substituiert sein kann,
R⁴ Wasserstoff, (C₁-C₈)-Alkyl, das durch Hydroxy, (C₁-C₄)-Alkoxy, (C₃-C₇)-Cycloalkyl, (C₆-C₁₀)-Aryl, 5- oder 6-gliedriges gesättigtes oder partiell ungesättigtes Heterocyclyl mit bis zu drei Heteroatomen aus der Reihe N, O und/oder S oder 5- bis 10-gliedriges Heteroaryl mit bis zu drei Heteroatomen aus der Reihe N, O und/oder S substituiert sein kann, oder (C₃-C₇)-Cycloalkyl, das durch Hydroxy oder (C₁-C₈)-Alkyl substituiert sein kann, bedeutet,
R⁵ (C₁-C₄)-Alkyl oder (C₁-C₄)-Alkoxy, die ein- oder zweifach, unabhängig voneinander, durch Hydroxy, (C₁-C₄)-Alkoxy, (C₃-C₇)-Cycloalkyl, (C₆-C₁₀)-Aryl oder 5- bis 10-gliedriges Heteroaryl mit bis zu drei Heteroatomen aus der Reihe N, O und/oder S substituiert sein können, wobei Aryl und Heteroaryl ihrerseits durch Halogen, (C₁-C₄)-Alkyl, (C₁-C₄)-Alkoxy, Amino, Mono- oder Di-(C₁-C₄)-alkylamino, Nitro, Cyano, Trifluormethyl oder Hydroxy substituiert sein können, oder (C₂-C₄)-Alkenyl bedeutet,
R⁶ (C₁-C₈)-Alkyl, das durch Hydroxy, (C₁-C₄)-Alkoxy, (C₃-C₇)-Cycloalkyl, (C₂-C₄)-Alkenyl, -CO-O-R⁸, (C₆-C₁₀)-Aryl oder 5- bis 10-gliedriges Heteroaryl mit bis zu drei Heteroatomen aus der Reihe N, O und/oder S substituiert sein kann wobei Aryl und Heteroaryl ihrerseits durch Halogen, (C₁-C₄)-Alkyl, (C₁-C₄)-Alkoxy, Amino, Mono- oder Di-(C₁-C₄)-alkylamino, Nitro, Cyano, Trifluormethyl oder Hydroxy substituiert sein können, (C₃-C₇)-Cycloalkyl oder -CO-O-R⁸ bedeutet,
worin
R⁸ Wasserstoff, (C₁-C₈)-Alkyl, das durch Hydroxy oder (C₁-C₄)-Alkoxy substituiert sein kann, (C₃-C₇)-Cycloalkyl oder (C₆-C₁₀)-Aryl, das bis zu dreifach, unabhängig voneinander, durch Halogen, Nitro, (C₁-C₄)-Alkoxy, Carboxyl, (C₁-C₄)-Alkoxycarbonyl oder Mono- oder Di-(C₁-C₄)-alkylamino substituiert sein kann, bedeutet,
oder
R⁵ und R⁶ gemeinsam mit dem Kohlenstoffatom, an das sie gebunden sind, einen 3-bis 7-gliedrigen, gesättigten oder partiell ungesättigten Ring bilden, der ein oder zwei Heteroatome aus der Reihe N, O und/oder S im Ring enthalten kann und der ein- bis dreifach, unabhängig voneinander, durch Oxo, Fluor, Chlor, Brom, Hydroxy, (C₁-C₆)-Alkyl oder (C₁-C₆)-Alkoxy substituiert sein kann,
und ihre Salze, Hydrate, Hydrate der Salze und Solvate.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die Tautomeren der Verbindungen der Formel (I).

Salze der Verbindungen der Formel (I) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem können die erfindungsgemäßen Verbindungen auch als Prodrugs vorliegen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:
Halogen steht im Allgemeinen für Fluor, Chlor, Brom oder Iod. Bevorzugt sind Fluor, Chlor oder Brom. Ganz besonders bevorzugt sind Fluor oder Chlor. (C₁-C₈)-Alkyl, (C₁-C₆)-Alkyl bzw. (C₁-C₄)-Alkyl steht im Allgemeinen für einen geradkettigen oder verzweigten Alkylrest mit 1 bis 8, 1 bis 6 bzw. 1 bis 4 Kohlenstoffatomen. Bevorzugt ist ein geradkettiger oder verzweigter Alkylrest mit 1 bis 6 Kohlenstoffatomen. Besonders bevorzugt ist ein geradkettiger oder verzweigter Alkylrest mit 1 bis 4 Kohlenstoffatomen. Beispielsweise seien genannt: Methyl, Ethyl, n-Propyl, Isopropyl, n-Butyl, sec-Butyl, Isobutyl und tert.-Butyl.
(C₂-C₄)-Alkenyl stehen im Allgemeinen für einen geradkettigen oder verzweigten Alkenylrest mit 2 bis 4 Kohlenstoffatomen. Beispielsweise seien genannt: Vinyl, Allyl, Isopropenyl und n-But-2-en-1-yl.
(C₂-C₄)-Alkinyl steht im Allgemeinen für einen geradkettigen oder verzweigten Alkinylrest mit 2 bis 4 Kohlenstoffatomen. Beispielsweise seien genannt: Ethinyl, n-Prop-2-in-1-yl und n-But-2-in-1-yl.
(C₁-C₈)-Alkoxy, (C₁-C₆)-Alkoxy bzw. (C₁-C₄)-Alkoxy steht im Allgemeinen für einen geradkettigen oder verzweigten Alkoxyrest mit 1 bis 8, 1 bis 6 bzw. 1 bis 4 Kohlenstoffatomen. Bevorzugt ist ein geradkettiger oder verzweigter Alkoxyrest mit 1 bis 6 Kohlenstoffatomen. Besonders bevorzugt ist ein geradkettiger oder verzweigter Alkoxyrest mit 1 bis 4 Kohlenstoffatomen. Beispielsweise seien genannt: Methoxy, Ethoxy, n-Propoxy, Isopropoxy, n-Butoxy, sec-Butoxy, Isobutoxy, tert.-Butoxy.
(C₁-C₄)-Alkoxycarbonyl steht im Allgemeinen für einen geradkettigen oder verzweigten Alkoxyrest mit 1 bis 4 Kohlenstoffatomen, der über eine Carbonylgruppe verknüpft ist. Beispielsweise seien genannt: Methoxycarbonyl, Ethoxycarbonyl, n-Propoxycarbonyl, Isopropoxycarbonyl und t-Butoxycarbonyl.
(C₁-C₄)-Alkanoyloxy steht im Allgemeinen für einen geraden oder verzweigten Alkylrest mit 1 bis 4 Kohlenstoffatomen, der in der 1-Position ein doppelt gebundenes Sauerstoffatom trägt und in der 1-Position über ein weiteres Sauerstoffatom verknüpft ist. Beispielsweise seien genannt: Acetoxy, Propionoxy, n-Butyroxy und i-Butyroxy.
Mono- oder Di-(C₁-C₄)-alkylamino steht im Rahmen der Erfindung für eine AminoGruppe mit einem oder mit zwei gleichen oder verschiedenen geradkettigen oder verzweigten Alkylsubstituenten, die jeweils 1 bis 4 Kohlenstoffatome aufweisen. Beispielsweise seien genannt: Methylamino, Ethylamino, n-Propylamino, Isopropylamino, t-Butylamino, *N,N*-Dimethylamino, *N,N*-Diethylamino, *N*-Ethyl-*N-*methylamino, *N*-Methyl-*N*-n-propylamino, *N*-Isopropyl-*N*-n-propylamino und *N*-t-Butyl-*N*-methylamino.
(C₃-C₇)-Cycloalkyl bzw. (C₃-C₆)-Cycloalkyl steht im Allgemeinen für einen cyclischen Alkylrest mit 3 bis 7 bzw. 3 bis 6 Kohlenstoffatomen. Bevorzugt sind cyclische Alkylreste mit 3 bis 6 Kohlenstoffatomen. Beispielsweise seien genannt: Cyclopropyl, Cyclobutyl, Cyclopentyl oder Cyclohexyl.
(C₆-C₁₀)-Aryl steht im Allgemeinen für einen aromatischen Rest mit 6 bis 10 Kohlenstoffatomen. Bevorzugte Arylreste sind Phenyl und Naphthyl.
(C₆-C₁₀)-Aryloxy steht im Allgemeinen für einen wie zuvor definierten aromatischen Rest, der über ein Sauerstoffatom verknüpft ist.
5- bis 10-gliedriges Heteroaryl mit bis zu 3 Heteroatomen aus der Reihe N, O und/oder S steht im Allgemeinen für einen mono- oder bicyclischen, gegebenenfalls benzokondensierten Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyrimidyl, Pyridazinyl, Pyrazinyl, Thienyl, Furyl, Pyrrolyl, Pyrazolyl, Imidazolyl, Triazolyl, Thiazolyl, Oxazolyl, Oxdiazolyl, Isoxazolyl, Benzofuranyl, Benzothienyl oder Benzimidazolyl. Aus dieser Definition leiten sich analog die entsprechenden Heteroaromaten mit weniger Heteroatomen wie z.B. mit einem oder 2 Heteroatomen aus der Reihe N, O und/oder S oder geringerer Ringgröße wie z.B. 5- oder 6-gliedriges Heteroaryl ab. Im Allgemeinen gilt, dass 5-oder 6-gliedrige aromatische Heterocyclen mit einem oder 2 Heteroatomen aus der Reihe N, O und/oder S bevorzugt sind. Beispielsweise seien genannt: Pyridyl, Pyrimidyl, Pyridazinyl, Furyl, Imidazolyl oder Thienyl.
5- bis 7-gliedriger Heterocyclus steht im Allgemeinen für einen gesättigten oder teilweise ungesättigten, gegebenenfalls benzokondensierten Heterocyclus mit bis zu 3 Heteroatomen aus der Reihe N, O und/oder S. Beispielsweise seien genannt: Tetrahydrofuryl, Pyrrolidinyl, Pyrrolinyl, Dihydropyridinyl, Piperidinyl, Piperazinyl, Morpholinyl, Thiomorpholinyl, Hexahydropyranyl. Aus dieser Definition leiten sich analog die entsprechenden Heterocyclen mit weniger Heteroatomen wie z.B. mit einem oder 2 Heteroatomen aus der Reihe N, O und/oder S oder geringerer Ringgröße wie z.B. 5-oder 6-gliedriges Heterocyclyl ab.Bevorzugt sind gesättigte Heterocyclen mit bis zu 2 Heteroatomen aus der Reihe N, O und/oder S, insbesondere Piperidinyl, Piperazinyl, Morpholinyl und Pyrrolidinyl.

### Bevorzugt sind Verbindungen der Formel (I),

worin
- R¹ und R²: unabhängig voneinander, Wasserstoff, (C₁-C₆)-Alkyl, (C₁-C₆)-Alkoxy, das durch Hydroxy, (C₁-C₄)-Alkoxy, (C₁-C₄)-Alkanoyloxy oder Cyclopropyl substituiert sein kann, Wasserstoff, Hydroxy, Fluor, Chlor, Nitro oder -NH-C(O)-CH₃ bedeuten
oder
- R¹ und R²: an benachbarte Phenylringatome gebunden sind und für eine Gruppe -O-CH₂-O- oder -O-CH₂-CH₂-O- stehen,
- R³: Wasserstoff bedeutet,
- R⁴: Wasserstoff, (C₁-C₄)-Alkyl, das durch Hydroxy, (C₁-C₄)-Alkoxy oder Cyclo-propyl substituiert sein kann, oder Cyclopropyl bedeutet,
- R⁵: (C₁-C₄)-Alkyl, das ein- oder zweifach, unabhängig voneinander, durch (C₃-C₆)-Cycloalkyl, Phenyl, das seinerseits durch Fluor, Trifluormethyl oder (C₁-C₄)-Alkoxy substituiert sein kann, Pyridyl, Furyl oder Thienyl substituiert sein kann, oder (C₂-C₄)-Alkenyl bedeutet
und
- R⁶: (C₁-C₄)-Alkyl oder (C₁-C₄)-Alkoxycarbonyl bedeutet
oder
- R⁵ und R⁶: gemeinsam mit dem Kohlenstoffatom, an das sie gebunden sind, einen 3 bis 7 gliedrigen, gesättigten oder partiell ungesättigten Ring bilden, der ein Heteroatom aus der Reihe N, O oder S im Ring enthalten kann,
und ihre Salze, Hydrate, Hydrate der Salze und Solvate.
Besonders bevorzugt sind Verbindungen der Formel (I),
worin
- R¹: Wasserstoff, Chlor, Nitro, Methyl, Methoxy, Ethoxy, n-Propoxy, Isopropoxy, n-Butoxy, wobei die Alkoxyreste ihrerseits durch Hydroxy, Methoxy, Ethoxy, n-Propoxy, Isopropoxy, n-Butoxy, -O-C(O)-CH₃ oder Cyclopropyl substituiert sein können, oder NH-C(O)-CH₃ bedeutet,
- R²: Wasserstoff bedeutet
oder
- R¹ und R² an: benachbarte Phenylringatome gebunden sind und für eine Gruppe -O-CH₂-O- stehen,
- R³: Wasserstoff bedeutet,
- R⁴: Wasserstoff, Methyl, Ethyl, n-Propyl, Isopropyl, n-Butyl, wobei die Alkyl-reste ihrerseits durch Hydroxy, Methoxy, Ethoxy, n-Propoxy, Isopropoxy, n-Butoxy oder Cyclopropyl substituiert sein können, oder Cyclopropyl bedeutet,
- R⁵: Methyl, Ethyl, n-Propyl, Isopropyl, n-Butyl, wobei die Alkylreste ihrerseits ein- oder zweifach, unabhängig voneinander, durch Cyclopropyl, Phenyl, das seinerseits durch Fluor, Trifluormethyl oder Methoxy substituiert sein kann, Pyridyl, Furyl oder Thienyl substituiert sein können, Ethenyl, Propenyl oder Butenyl bedeutet
und
- R⁶: Methyl, Ethyl, n-Propyl, Isopropyl, n-Butyl, Methoxycarbonyl, Ethoxy-carbonyl, n-Propoxycarbonyl, Isopropoxycarbonyl, n-Butoxycarbonyl oder Isobutoxycarbonyl bedeutet
oder
- R⁵ und R⁶: gemeinsam mit dem Kohlenstoffatom, an das sie gebunden sind, einen Cyclopropyl-, Cyclobutyl, Cyclopentyl- oder Cyclohexylring bilden
und ihre Salze, Hydrate, Hydrate der Salze und Solvate.

Ebenfalls besonders bevorzugt sind Verbindungen der Formel (I), worin R¹ und R² an benachbarte Phenylringatome gebunden sind, die sich in para- und meta-Position zur Anknüpfungsstelle des Pyridinrings befinden, und für eine Gruppe -O-CH₂-O-stehen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen, beliebig kombiniert werden. Sie gelten für die Endprodukte sowie für die Vor- und Zwischenprodukte entsprechend.

Die vorliegende Erfindung betrifft weiterhin ein Verfahren zur Herstellung der Verbindungen der Formel (I), dadurch gekennzeichnet, dass man entweder
[A] Verbindungen der Formel (II) 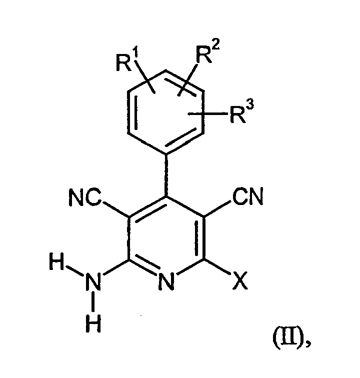 in welcher
   R¹, R² und R³ die oben angegebene Bedeutung haben und X für eine geeignete Abgangsgruppe wie beispielsweise Chlor, Brom, Methylthio oder Phenylthio steht,
   zunächst mit Malonsäureamidethylester (III)

   H₂N-C(O)- CH₂ - C(O)-O-C₂H₅ (III),

   zu Verbindungen der Formel (IV) 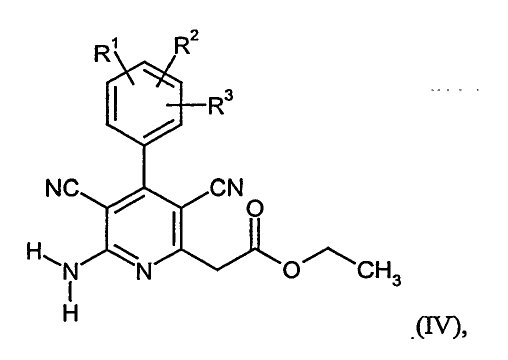 in welcher
   R¹, R² und R³ die oben angegebene Bedeutung haben,
   umsetzt
   und dann mit Verbindungen der Formel (V)

   R⁵- Y (V),

   in welcher
   R⁵ die oben angegebene Bedeutung hat und Y für eine geeignete Abgangsgruppe wie beispielsweise Chlor, Brom oder Jod steht,
   zu Verbindungen der Formel (I) umsetzt,
   in welcher
   R¹, R², R³, R⁴ und R⁵ die oben angegebene Bedeutung haben und R⁶ für einen Rest -C(O)-O-C₂H₅ steht,
   und gegebenenfalls anschließend mit Verbindungen der Formel (VI)

   R⁸ - OH (VI),

   in welchen R⁸ die oben angegebene Bedeutung hat,
   zu Verbindungen der Formel (I) umsetzt,
   in welcher
   R⁶ für einen Rest -C(O)-O-R⁸ steht und R¹, R², R³, R⁴, R⁵ und R⁸ die oben angegebene Bedeutung haben,
   oder
[B] Verbindungen der Formel (II)
   in einem inerten Lösungsmittel in Gegenwart eines Katalysators mit Grignardverbindungen der Formel (VII) 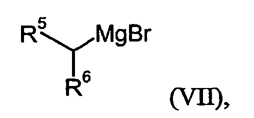
in welcher
R⁵ und R⁶ die oben angegebene Bedeutung haben,
zu Verbindungen der Formel (I) umsetzt,
in welcher
R¹, R², R³, R⁵ und R⁶ die oben angegebene Bedeutung haben und R⁴ für Wasserstoff steht,
und gegebenfalls anschließend mit Verbindungen der Formel (VIII)

R⁴ - Y' (VIII),

in welcher
R⁴ die oben angegebene Bedeutung und Y' die Bedeutung von Y hat,
umsetzt.

Das erfindungsgemäße Verfahren kann durch folgendes Formelschema beispielhaft erläutert werden:

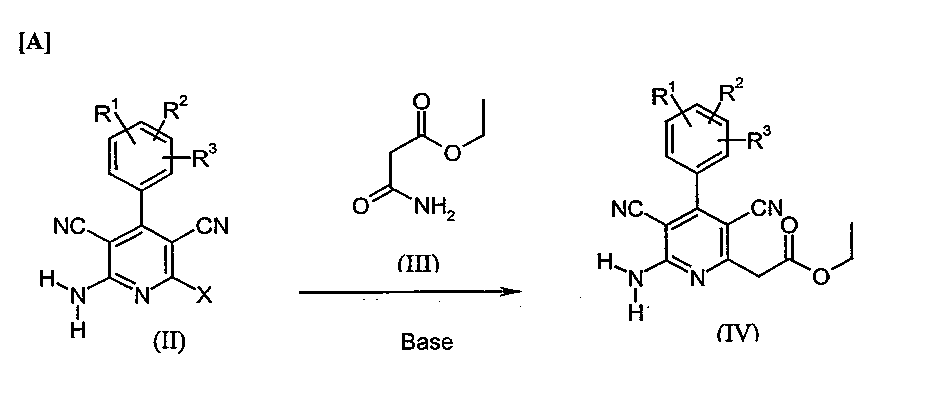

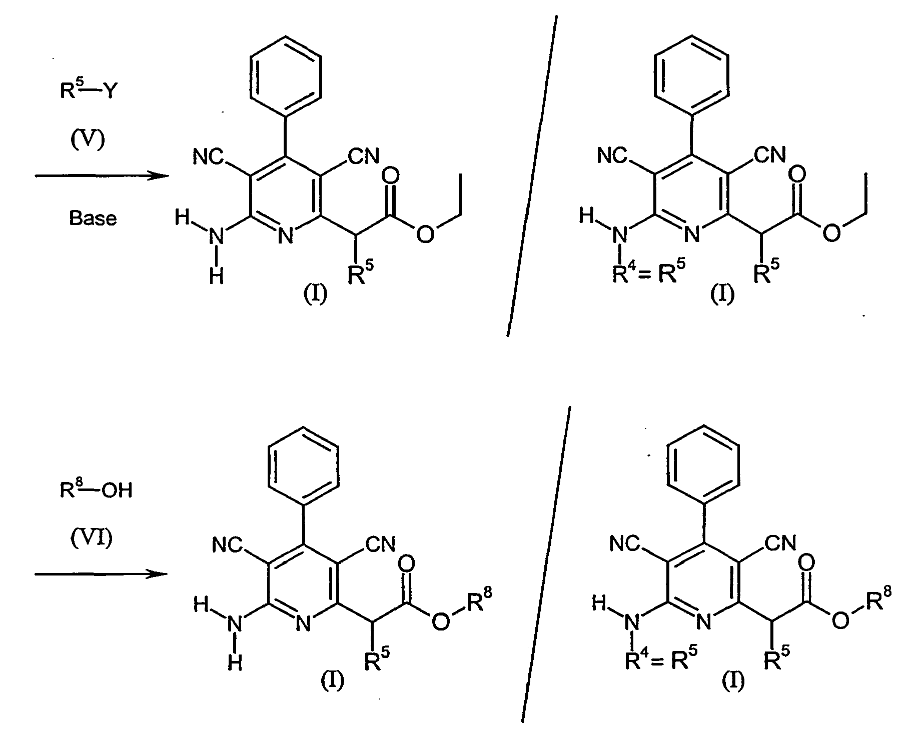

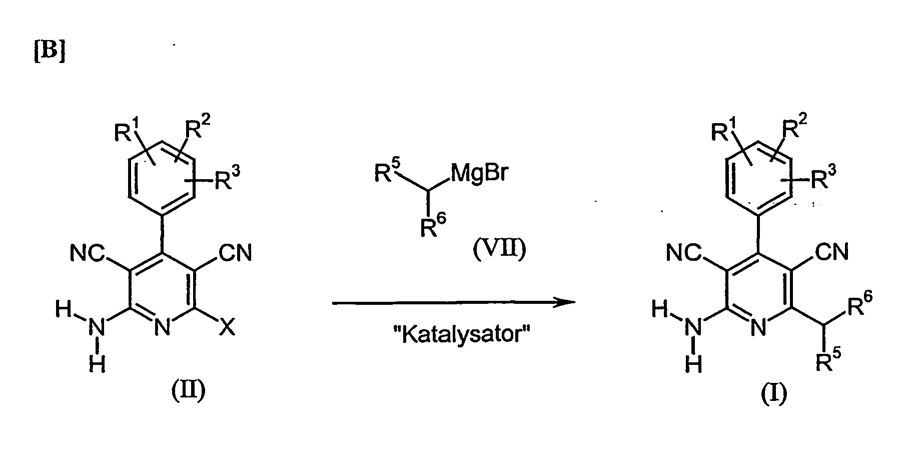

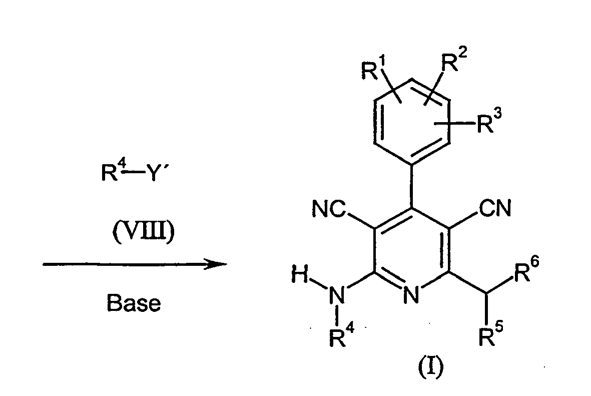

Für den ersten Verfahrensschritt [A]: (II) + (III) → (IV) eignen sich als Lösungsmittel organische Lösungsmittel, die sich unter den Reaktionsbedingungen nicht verändern. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Ebenso ist es möglich, Gemische der zuvor genannten Lösungsmittel einzusetzen. Bevorzugt ist DMF.

Als Basen eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium-bis-(trimethylsilyl)amid oder Lithiumdiisopropylamid, metallorganische Verbindungen wie Butyllithium oder Phenyl-lithium, oder auch das Natrium- oder Kaliumsalz der jeweiligen Verbindung der allgemeinen Formel (VI) selbst. Bevorzugt sind Kalium-tert.-butylat und Kaliumcarbonat.

Die Base kann hierbei in einem Verhältnis von 1 bis 10 mol, bevorzugt in einem Verhältnis von 1 bis 5 mol, insbesondere in einem Verhältnis von 1 bis 4 mol Base zu 1 mol der Verbindung (II) eingesetzt werden.

Die Reaktion erfolgt im Allgemeinen in einem Temperaturbereich von -78°C bis +150°C, bevorzugt im Bereich von +20°C bis +80°C, insbesondere bei +20°C bis +60°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im Allgemeinen mit einem Überschuss an Verbindung (III), bevorzugt in einem Verhältnis von 1,5 bis 8 mol der Verbindung (III) zu 1 mol der Verbindung (II).

Im zweiten Verfahrensschritt [A]: (IV) + (V) → (I) kann unter den Reaktionsbedingungen gegebenenfalls ein Produktgemisch entstehen, wobei neben dem Kohlenstoffatom in α-Position zur Esterfunktion auch das Stickstoffatom der Aminopyridineinheit alkyliert wird. Auf diese Weise werden Verbindungen der Formel (I) erhalten, in welcher der Substituent R⁴ entweder für Wasserstoff steht oder die Bedeutung von R⁵ hat. Die verschiedenen Produkte können chromatographisch getrennt werden.

Als Lösungsmittel für diese Reaktion eignen sich organische Lösungsmittel, die unter den Reaktionsbedingungen inert sind. Hierzu gehören Ketone wie Aceton und Methylethylketon, acyclische und cyclische Ether wie Diethylether, 1,2-Dimethoxyethan oder Tetrahydrofuran, Ester wie Essigsäureethylester oder Essigsäure-butylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Ebenso ist es möglich, Gemische der zuvor genannten Lösungsmittel einzusetzen. Bevorzugt ist DMF.

Als Basen eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium-bis-(trimethylsilyl)amid oder Lithiumdiisopropylamid, metallorganische Verbindungen wie Butyllithium oder Phenyl-lithium, Amine wie Triethylamin oder Pyridin, oder auch das Natrium- oder Kaliumsalz der jeweiligen Verbindung der allgemeinen Formel (IV) selbst. Bevorzugt sind Kalium-tert.-butylat und Kaliumcarbonat.

Die Base kann hierbei in einem Verhältnis von 1 bis 10 mol, bevorzugt in einem Verhältnis von 1 bis 5 mol, insbesondere in einem Verhältnis von 1 bis 4 mol Base zu 1 mol der Verbindung (IV) eingesetzt werden.

Die Reaktion erfolgt im Allgemeinen in einem Temperaturbereich von -78°C bis +120°C, bevorzugt im Bereich von +20°C bis +100°C, insbesondere bei +20°C bis +80°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im Allgemeinen mit einer äquivalenten Menge oder mit einem Überschuss an Verbindung (V), bevorzugt in einem Verhältnis von 1 bis 5 mol der Verbindung (V) zu 1 mol der Verbindung (IV).

Für den gegebenenfalls erfolgenden dritten Verfahrensschritt [A]: (I) + (VI) → (I) eignen sich als Lösungsmittel organische Lösungsmittel, die unter den Reaktionsbedingungen inert sind. Bevorzugt wird in einem Überschuss an Alkohol (VI) als Lösungsmittel gearbeitet.

Die Reaktion erfolgt im Allgemeinen in einem Temperaturbereich von -78°C bis +120°C, bevorzugt im Bereich von +20°C bis +100°C, insbesondere bei +30°C bis +80°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im Allgemeinen mit einem großen Überschuss an Verbindung (VI), die gleichzeitig als Lösungsmittel der Reaktion dient.

Die Umsetzung erfolgt im Allgemeinen in Gegenwart eines basischen Katalysators. Als basische Katalysatoren eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium-bis-(trimethylsilyl)amid oder Lithiumdiisopropylamid, metallorganische Verbindungen wie Butyllithium oder Phenyl-lithium, Amine wie Triethylamin oder Pyridin, oder auch das Natrium- oder Kaliumsalz der jeweiligen Verbindung der allgemeinen Formel (VI) selbst. Weiterhin eignen sich als basische Katalysatoren basiche Reduktionsmittel wie z.B. Natriumborhydrid oder Kaliumborhydrid. Bevorzugt sind Natriumborhydrid, Kalium-tert.-butylat und Kaliumcarbonat.

Für den ersten Verfahrensschritt [B]: (II) + (VII) → (I) eignen sich als Lösungsmittel organische Lösungsmittel, die unter den Reaktionsbedingungen inert sind. Hierzu gehören acyclische und cyclische Ether wie Diethylether, 1,2-Dimethoxyethan oder Tetrahydrofuran, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Pyridin oder Dimethylsulfoxid (DMSO). Ebenso ist es möglich, Gemische der zuvor genannten Lösungsmittel einzusetzen. Bevorzugt sind Diethylether oder Tetrahydrofuran.

Die Reaktion erfolgt im Allgemeinen in einem Temperaturbereich von -78°C bis +120°C, bevorzugt im Bereich von +20°C bis +60°C, insbesondere bei +40°C bis +60°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Als Katalysator können Nickel(II)-Komplexe wie z.B. 1,3-Bis(diphenylphosphino)-propan-dichlornickel(II) oder Bis(triphenylphosphin)dichlornickel(II) verwendet werden (siehe Chemistry Letters 1447-1450 (1979) ). Der Katalysator wird in einem Verhältnis von 0,001 bis 0,1 mol, bevorzugt in einem Verhältnis von 0,03 bis 0,1 mol Katalysator zu 1 mol der Verbindung (II) eingesetzt.

Die Umsetzung erfolgt im Allgemeinen mit einer äquivalenten Menge oder mit einem Überschuß an Verbindung (VII), bevorzugt in einem Verhältnis von 2 bis 8 mol der Verbindung (VII), besonders bevorzugt in einem Verhältnis von 2 bis 4 mol der Verbindung (VII) zu 1 mol der Verbindung (II).

Für den gegebenenfalls erfolgenden zweiten Verfahrensschritt [B]: (I) + (VIII) → (I) eignen sich als Lösungsmittel organische Lösungsmittel, die unter den Reaktionsbedingungen inert sind. Hierzu gehören Ketone wie Aceton und Methylethylketon, acyclische und cyclische Ether wie Diethylether, 1,2-Dimethoxyethan oder Tetrahydrofuran, Ester wie Essigsäureethylester oder Essigsäure-butylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Ebenso ist es möglich, Gemische der zuvor genannten Lösungsmittel einzusetzen. Bevorzugt ist DMF.

Als Basen eignen sich die üblichen anorganischen oder organischen Basen. Hierzu gehören Alkalihydroxide wie beispielsweise Natrium- oder Kaliumhydroxid, Alkalicarbonate wie Natrium- oder Kaliumcarbonat oder Natrium- oder Kaliumhydrogencarbonat, Kalium-tert.-butylat, Natriumhydrid, Amide wie Natriumamid, Lithium-bis-(trimethylsilyl)amid oder Lithiumdiisopropylamid, metallorganische Verbindungen wie Butyllithium oder Phenyl-lithium, Amine wie Triethylamin oder Pyridin, oder auch das Natrium- oder Kaliumsalz der jeweiligen Verbindung der allgemeinen Formel (IV) selbst. Bevorzugt sind Kalium-tert.-butylat und Kaliumcarbonat.

Die Base kann hierbei in einem Verhältnis von 1 bis 10 mol, bevorzugt in einem Verhältnis von 1 bis 5 mol, insbesondere in einem Verhältnis von 1 bis 4 mol Base zu 1 mol der Verbindung (I) eingesetzt werden.

Die Reaktion erfolgt im Allgemeinen in einem Temperaturbereich von -78°C bis +120°C, bevorzugt im Bereich von +20°C bis +100°C, insbesondere bei +20°C bis +80°C.

Die Umsetzung kann bei normalem, erhöhtem oder erniedrigtem Druck durchgeführt werden, z.B. im Bereich von 0,5 bis 5 bar. Im Allgemeinen arbeitet man bei Normaldruck.

Die Umsetzung erfolgt im allgemeinen mit einer äquivalenten Menge oder mit einem Überschuss an Verbindung (VIII), bevorzugt in einem Verhältnis von 1 bis 5 Mol der Verbindung (VIII) zu 1 mol der Verbindung (I).

Die Verbindungen der Formel (II) sind dem Fachmann bekannt oder können in Analogie zu literaturbekannten Methoden hergestellt werden [siehe beispielsweise J.M. Quintela, J.L. Soto, Anales de Quimica 79, 368-372 (1983)].

Die Verbindungen der Formel (III), (V), (VI) und (VIII) sind kommerziell erhältlich, dem Fachmann bekannt oder nach literaturüblichen Methoden herstellbar.

Die Verbindungen der Formel (VII) sind dem Fachmann bekannt oder können in Analogie zu literaturbekannten Methoden hergestellt werden [siehe beispielsweise Organikum, 18. ber. Ausg., Deutscher Verlag der Wissenschaften, Berlin 1990, Seite 499].

Überraschenderweise zeigen die Verbindungen der Formel (I) ein nicht vorhersehbares, wertvolles pharmakologisches Wirkspektrum und sind daher insbesondere zur Prophylaxe und/oder Behandlung von Erkrankungen geeignet.

Die Verbindungen der Formel (I) sind zur Prophylaxe und/oder Behandlung einer ganzen Reihe von Erkrankungen geeignet, so beispielsweise insbesondere von Erkrankungen des Herzkreislaufsystems (kardiovaskulären Erkrankungen).

Im Sinne der vorliegenden Erfindung sind unter Erkrankungen des Herz-Kreislauf-Systems bzw. kardiovaskulären Erkrankungen beispielsweise insbesondere die folgenden Erkrankungen zu verstehen: Koronare Herzkrankheit, Hypertonie (Bluthochdruck), Restenose wie z.B. Restenose nach Ballondilatation von peripheren Blutgefäßen, Arteriosklerose, Tachykardien, Arrhythmien, periphere und kardiale Gefäßerkrankungen, stabile und instabile Angina pectoris und Vorhofflimmern.

Weiterhin eignen sich die Verbindungen der Formel (I) beispielsweise insbesondere auch zur Reduktion des von einem Infarkt betroffenen Myokardbereichs.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Substanzen der Formel (I) zur Herstellung von Arzneimitteln und pharmazeutischen Zusammensetzungen zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Substanzen der Formel (I).

Die pharmazeutische Wirksamkeit der zuvor genannten Verbindungen der Formel (I) lässt sich insbesondere durch ihre Wirkung als selektive Liganden an A1-Adenosinrezeptoren erklären.

Als "selektiv" werden im Rahmen der vorliegenden Erfindung solche Adenosin-rezeptor-Liganden bezeichnet, bei denen einerseits eine deutliche Wirkung an einem oder mehreren Adenosin-Rezeptor-Subtypen und andererseits keine oder eine deutlich schwächere Wirkung an einem oder mehreren anderen Adenosinrezeptor-Subtypen zu beobachten ist, wobei bezüglich der Testmethoden für die Wirk-Selektivität Bezug genommen wird auf die im Abschnitt A. II. beschriebenen Testmethoden.

Ein Vorteil der erfindungsgemäßen Verbindungen der Formel (I) ist, dass sie gegenüber Adenosinrezeptor-Liganden des Standes der Technik selektiver wirken.

Die Rezeptorselektivität kann bestimmt werden durch die biochemische Messung des intrazellulären Botenstoffes cAMP in den transfizierten Zellen, die spezifisch nur einen Subtyp der Adenosinrezeptoren exprimieren. Im Falle von A1-Agonisten (Kopplung bevorzugt über Gi-Proteine) wird dabei eine Abnahme des intrazellulären cAMP-Gehaltes festgestellt, unter Bedingungen bei denen die intrazelluläre cAMP Konzentration durch Stimulation der Adenylatzyklase deutlich erhöht würde. Im Falle von A1-Antagonisten wird dagegen ein Anstieg des intrazellulären cAMP-Gehaltes nach vergleichbarer Vorstimulation der Adenylatzyklase plus Stimulation mit Adenosin oder Adenosin ähnlichen Substanzen beobachtet.

So eignen sich Verbindungen der Formel (I), die selektiv an Adenosin-A1-Rezeptoren binden, bevorzugt zur Myokard-Protektion und zur Prophylaxe und/oder Behandlung von Tachykardien, Vorhof-Arrhythmien, Herzinsuffizienz, Herzinfarkt, akutem Nierenversagen, Diabetes, Schmerzzuständen sowie zur Wundheilung.

Weiterer Gegenstand der vorliegenden Erfindung sind Arzneimittel und pharmazeutische Zusammensetzungen, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal, wie z.B. bei Implantaten oder Stents, oder äußerlich wie z.B. transdermal. Bei der parenteralen Applikation sind insbesondere intravenöse, intramuskuläre, subkutane Applikation zu nennen, z.B. als subkutanes Depot. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im Allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten auch allgemein übliche Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Im Allgemeinen hat es sich als vorteilhaft erwiesen, bei parenteraler Applikation Mengen von etwa 0,1 bis etwa 10 000 µg/kg, vorzugsweise etwa 1 bis etwa 1000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,1 bis etwa 10 mg/kg, vorzugsweise etwa 0,5 bis etwa 5 mg/kg, insbesondere etwa 1 bis etwa 4 mg/kg Körpergewicht.

In Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt, kann es gegebenenfalls erforderlich sein, von den genannten Mengen abzuweichen.

Die vorliegende Erfindung wird an den folgenden Beispielen veranschaulicht, die die Erfindung jedoch keinesfalls beschränken.

### A. Bewertung der physiologischen Wirksamkeit

### I. Nachweis der kardiovaskulären Wirkung

### Langendorff-Herz der Ratte:

Narkotisierten Ratten wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### II. Nachweis der Rezeptorselektivität

### a) Adenosin-A1-, A2a-, A2b- und A3-Rezeptorselektivität

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des intrazellulären cAMP-Gehaltes in diesen Zellen mit einem konventionellen radioimmunologischen Assay (cAMP-RIA).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die nicht selektiv, aber mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

Die Adenosin-Rezeptoren A1 und A3 sind an ein Gi-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), die nicht selektiv, aber mit hoher Affinität an alle Adenosinrezeptor-Subtypen bindet und eine antagonistische Wirkung besitzt (Müller, C.E., Stein, B., Adenosine receptor antagonists: structures and potential therapeutic applications, Current Pharmaceutical Design, 2 (1996), 501-530).

### b) Adenosin-A1-, A2a-, A2b- Rezeptorselektivität

Zellen der permanenten Linie CHO (Chinese Hamster Ovary) werden stabil mit der cDNA für die Adenosin-Rezeptor-Subtypen A1, A2a, A2b transfiziert. Die Adenosin A1 Rezeptoren sind über Gᵢ-Proteine und die Adenosin A2a und A2b Rezeptoren über Gs-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luciferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanztest-Screening wird das folgende Testprotokoll verwendet:

Die Stammkulturen wird in DMEM/F12 Medium mit 10 % FCS (fötales Kälberserum) bei 37°C unter 5 % CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37°C angezogen. Dann wird das Medium durch eine physiologische Kochsalzlösung (130 mM NaCl, 5 mM KCl, 2 mM CaCl₂, 20 mM HEPES, 1 mM MgCl₂●6H₂O, 5 mM NaHCO₃, pH 7,4) ersetzt. Die in DMSO gelösten Substanzen werden 3 mal 1:10 mit dieser physiologischen Kochsalzlösung verdünnt und zu den Testkulturen pipettiert (maximale DMSO-Endkonzentration im Testansatz: 0,5 %) So erhält man Substanzendkonzentrationen von beispielsweise 5 µM bis 5 nM. 10 Minuten später wird Forskolin zu den A1 Zellen zugegeben und anschließend werden alle Kulturen für 4 Stunden bei 37°C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50% aus Luciferase Substrat Lösung (2,5 mM ATP, 0,5 mM Luciferin, 0,1 mM Coenzym A, 10 mM Tricin, 1,35 mM MgSO₄, 15 mM DTT, pH 7,8) zugegeben, ca. 1 Minute geschüttelt und die Luciferase-Aktivität mit einem Kamerasystem gemessen.

### B. Ausführungsbeispiele

### Verwendete Abkürzungen:

- Äq.: Äquivalente
- DMF: Dimethylformamid
- DMSO: Dimethylsulfoxid
- d. Th.: der Theorie
- HPLC: Hochdruck-, Hochleistungsflüssigchromatographie
- NMR: Kernresonanzspektroskopie
- RP: reversed phase
- THF: Tetrahydrofuran
- i. V.: im Vakuum

### Beispiel 1

### 2-[6-Amino-4-(1,3-benzodioxol-5-yl)-3,5-dicyano-2-pyridinyl]-propionsäure-ethylester

### Stufe 1

### [6-Amino-4-(1,3-benzodioxol-5-yl)-3,5-dicyano-2-pyridinyl]-essigsäureethylester

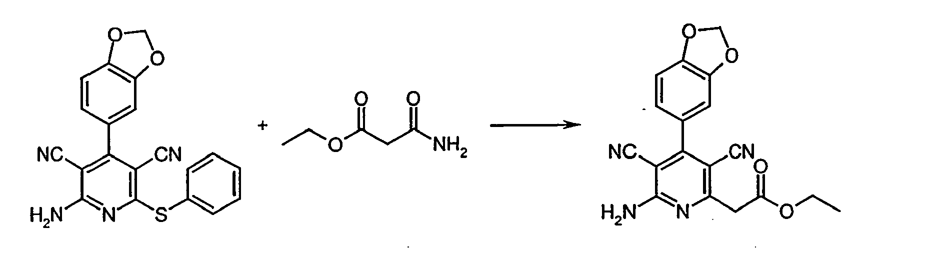

2-Amino-4-(1,3-benzodioxol-5-yl)-6-(phenylsulfanyl)-3,5-dicyano-pyridin (5,00 g, 13,43 mmol) [Herstellung analog zu J.M. Quintela, J.L. Soto, Anales de Quimica 79, 368-372 (1983)] wird mit Malonsäureamidethylester (4,23 g, 23,22 mmol) in absolutem DMF (30 ml) unter Argonatmosphäre vorgelegt. Nach Zugabe von Kalium-tert-butylat (3,01 g, 26,85 mmol) wird die Lösung für 22 Stunden bei Raumtemperatur gerührt. Der Ansatz wird in Wasser (300 ml) gegeben. Dann wird dreimal mit Essigsäureethylester (je 300 ml) extrahiert, die vereinigten organischen Phasen über Magnesiumsulfat getrocknet, filtriert und auf ca. 100 ml eingeengt. Die ausfallenden Kristalle werden abgesaugt.
Ausbeute: 3,5 g (74 % d.Th.)

¹H-NMR (200 MHz, DMSO-d₆): δ = 1.20 (t, 3H), 3.89 (s, 2H), 4.13 (q, 2H), 6.16 (s, 2H), 7.01 - 7.17 (m, 3H), 8.00 (bs, 2H).
ESI (positiv) ber. 350,33 gef. 351,166

### Stufe 2

### 2-[6-Amino-4-(1,3-benzodioxol-5-yl)-3,5-dicyano-2-pyridinyl]-propionsäure-ethylester

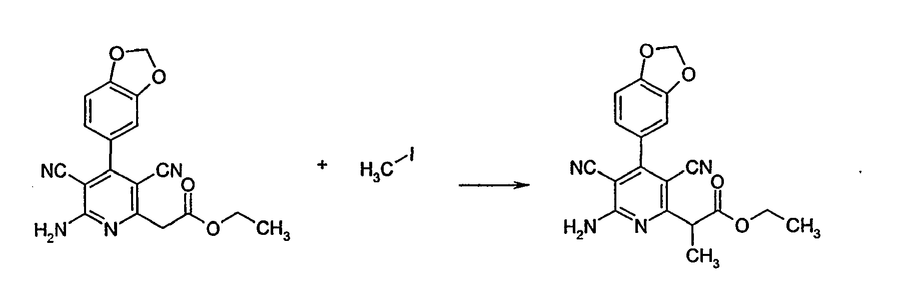

[6-Amino-4-(1,3-benzodioxol-5-yl)-3,5-dicyano-2-pyridinyl]-essigsäureethylester (Stufe 1) (2,00 g, 5,71 mmol) wird in 40 ml DMF vorgelegt. Es wird Natriumhydrid (0,37 g, 9,19 mmol) zugegeben und für 45 Minuten gerührt. Anschließend wird Methyliodid (0,40 ml, 6,39 mmol) zugegeben, wobei sich die gelbe Reaktionslösung orange färbt. Nach fünf Stunden werden weitere 0,2 ml Methyliodid zugegeben und über Nacht bei Raumtemperatur gerührt. Der Ansatz wird in Wasser (50 ml) gegeben, wobei eine Emulsion entsteht. Es wird dreimal mit Ether extrahiert. Die vereinigten organischen Phasen werden über Magnesiumsulfat getrocknet, filtriert und einrotiert. Das Gemisch wird mittels RP-HPLC mit einem Acetonitril/Wasser-Gradienten chromatographiert. Das Produkt fällt als farbloser Feststoff an.
Ausbeute: 0,58 g (28 % d.Th.)

¹H-NMR (200 MHz, DMSO-d₆): δ = 1.15 (t, 3H), 1.44 (d, 3H), 4.05 - 4.16 (m, 3H), 6.16 (s, 2H), 7.02 - 7.19 (m, 3H), 7.93 (bs, 2H).
ESI (positiv) ber. 364,36 gef. 364,997

Die entsprechenden Ethyl- bzw. Allyl-substituierten Verbindungen aus Beispiel 2 und 3 werden analog hergestellt:

### Beispiel 2

### 2-[6-Amino-4-(1,3-benzodioxol-5-yl)-3,5-dicyano-2-pyridinyl]-pent-4-ensäure-ethylester

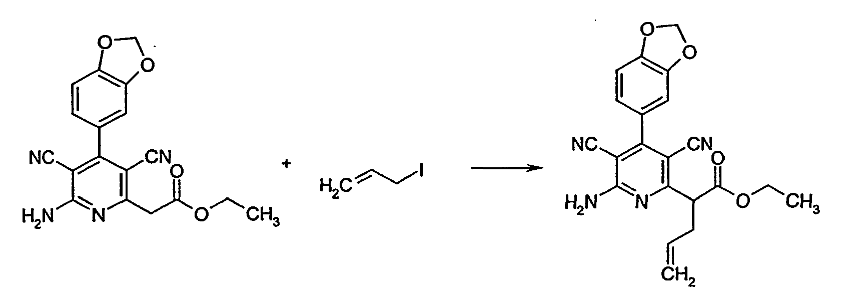

1 Äq. Allylbromid, 1.1 Äq. Natriumhydrid
Ausbeute: 71 % d.Th.

¹H-NMR (200 MHz, DMSO-d₆): δ = 1.11 -1.18 (t, 3H), 2.59 - 2.84 (m, 2H), 4.06 - 4.16 (m, 3H), 5.00 - 5.09 (m, 2H), 5.72 - 5.85 (m, 1H), 6.16 (s, 2H), 7.00 - 7.18 (m, 3H), 8.00 (bs, 2H).
ESI (positiv) ber. 390,4 gef. [M+H] 391,1

### Beispiel 3

### 2-[6-Amino-4-(1,3-benzodioxol-5-yl)-3,5-dicyano-2-pyridinyl]-buttersäure-ethylester

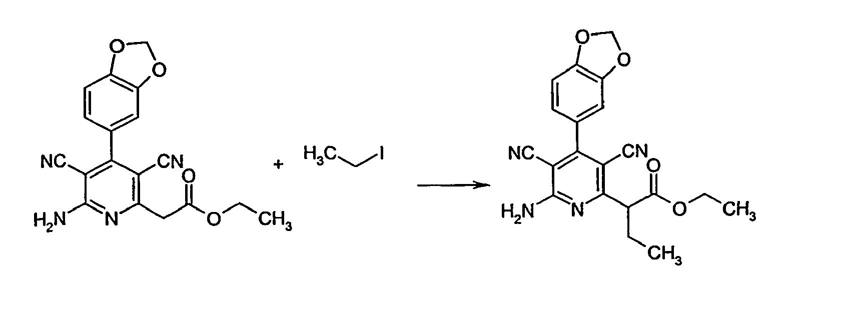

1,12 Äq. Ethyliodid, 1.61 Äq. Natriumhydrid.
Ausbeute: 37 % d.Th.
¹H-NMR (200 MHz, DMSO-d₆): δ = 0.88 (t, 2H), 1.81 - 2.15 (m, 2H), 3.63 (s, 3H), 3.92 - 3.99 (m, 1H), 6.16 (s, 2H), 7.02 - 7.20 (m, 3H), 7.98 (bs, 2H).
ESI (positiv) ber. 364,36 gef. 364,978

### Beispiel 4

### 2-[4-(1,3-Benzodioxol-5-yl)-3,5-dicyano-6-methylamino-2-pyridinyl]-propionsäureethylester

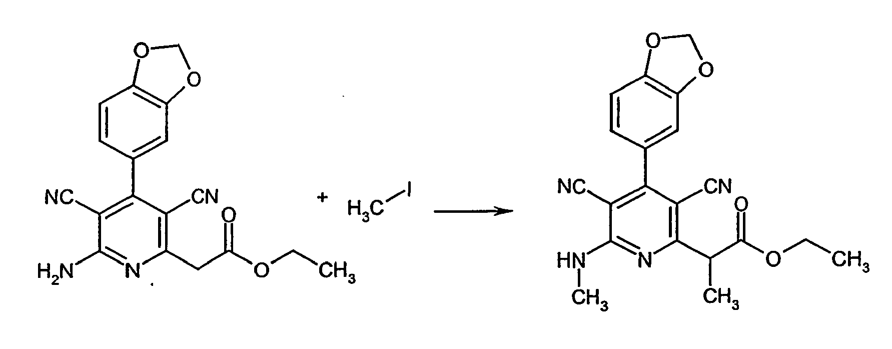

Die Verbindung entsteht bei der Umsetzung nach Beispiel 1, Stufe 2 und wird aus dem Rohgemisch heraus isoliert, indem das Gemisch mittels RP-HPLC mit einem Acetonitril/Wasser-Gradienten chromatographiert wird. Das Produkt fällt als farbloser Feststoff an.
Ausbeute: 1,1 g (51 % d.Th.).
ESI (positiv) ber. 378,39 gef. 378,3

### Beispiel 5

### 2-[4-(1,3-Benzodioxol-5-yl)-3,5-dicyano-6-ethylamino-2-pyridinyl]-buttersäure-ethylester

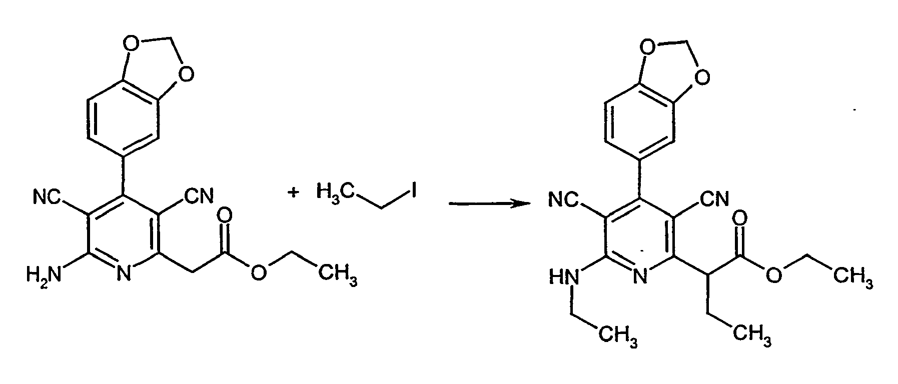

Die Verbindung entsteht bei der Umsetzung nach Beispiel 4 und wird aus dem Rohgemisch heraus isoliert, indem das Gemisch mittels RP-HPLC mit einem Acetonitril/Wasser-Gradienten chromatographiert wird.
Ausbeute: 13 % d.Th.
ESI (positiv) ber. 392,413 gef. [M+H] 393,2

### Beispiel 6

### 2-[6-Amino-4-(1,3-benzodioxol-5-yl)-3,5-dicyano-2-pyridinyl]-propionsäure-methylester

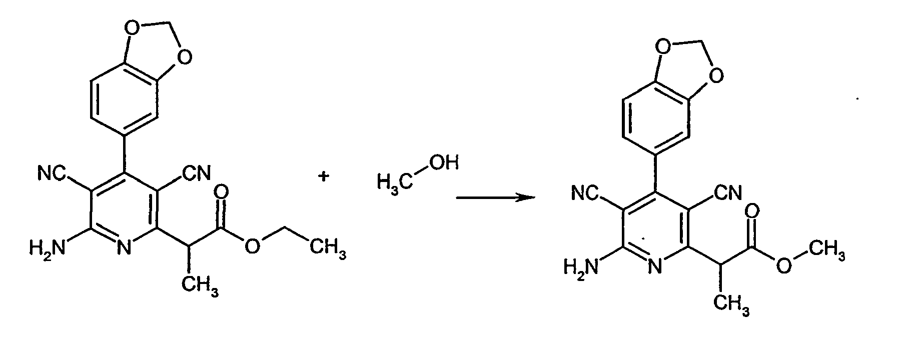

2-[6-Amino-4-(1,3-benzodioxol-5-yl)-3,5-dicyano-2-pyridinyl]-propionsäureethyl-ester (aus Beispiel 1) (50 mg, 0,137 mmol) wird in Methanol (2 ml) mit einer katalytischen Menge Natriumborhydrid für 1,5 Stunden unter Rückfluß erhitzt. Es werden 1N Salzsäure und gesättigte Kochsalzlösung zugegeben. Die wässrige Phasen werden zweimal mit Ethylacetat extrahiert und die vereinigten organischen Phasen über Magnesiumsulfat getrocknet, filtriert und am Rotationsverdampfer eingeengt. Die Isolierung des Produkts erfolgt mittels RP-HPLC (Kromasilsäule 250 * 20 mm, C18, 10 µm; Acetonitril/Wasser-Gradient: 3 Minuten 10 %, dann innerhalb 30 Minuten auf 80 %, Flussrate: 25 ml*min⁻¹).
Ausbeute: 24 mg (50 % d.Th.).
¹H-NMR (200 MHz, DMSO-d₆): δ = 1.45 (d, 3H), 3.64 (s, 3H), 4.16 (q, 1H), 6.16 (s, 2H), 7.07 - 7.20 (m, 3H), 7.98 (bs, 2H).
ESI (positiv) ber. 350,33 gef. [M+H] 351,139

Die im Folgenden beschriebenen Beispielverbindungen 7 bis 9 werden analog zu Beispiel 6 hergestellt, wobei Methanol gegen den entsprechenden Alkohol als Lösungsmittel ausgetauscht wird:

### Beispiel 7

### 2-[6-Amino-4-(1,3-benzodioxol-5-yl)-3,5-dicyano-2-pyridinyl]-propionsäure-butylester

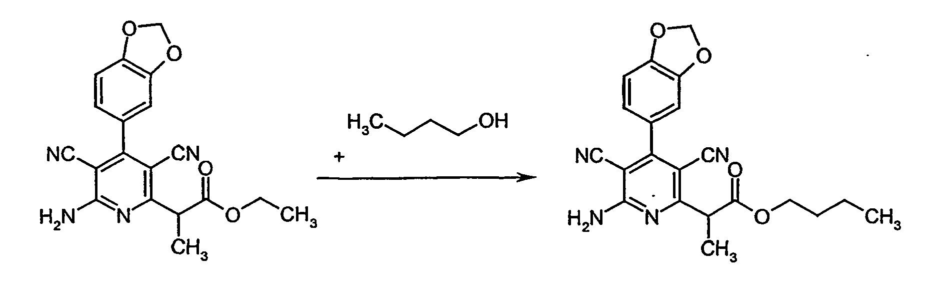

Ausbeute: 47 % d.Th.
¹H-NMR (200 MHz, DMSO-d₆): δ = 1.42 (d, 3H), 1.52 - 1,77 (m, 2H), 1.88 - 2.05 (m, 2H), 2.20 - 2.30 (m, 2H), 4.11 (q, 1H), 4.87 - 4.97 (m, 1H), 6.16 (s, 2H), 7.03 - 7.19 (m, 3H), 7.97 (bs, 2H).
ESI (positiv) ber. 390,4 gef. [M+H] 391,284

### Beispiel 8

### 2-[6-Amino-4-(1,3-benzodioxol-5-yl)-3,5-dicyano-2-pyridinyl]-propionsäure-isopropylester

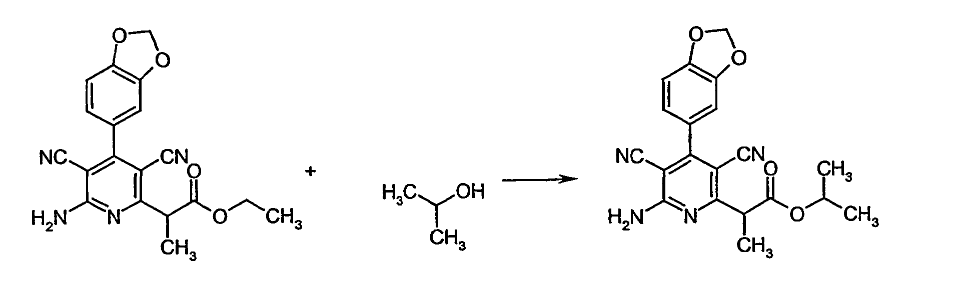

Ausbeute: 21 % d.Th.
¹H-NMR (200 MHz, DMSO-d₆): δ = 1.16 (dd, 6H), 1.42 (d, 3H), 4.08 (q, 1H), 4.92 - 4.96 (m, 1H), 6.16 (s, 2H), 7.02 - 7.18 (m, 3H), 7.95 (bs, 2H).
ESI (positiv) ber. 378,39 gef. [M+H] 379,26

### Beispiel 9

### 2-[4-(1,3-Benzodioxol-5-yl)-3,5-dicyano-6-methylamino-2-pyridinyl]-propion-säureisobutylester

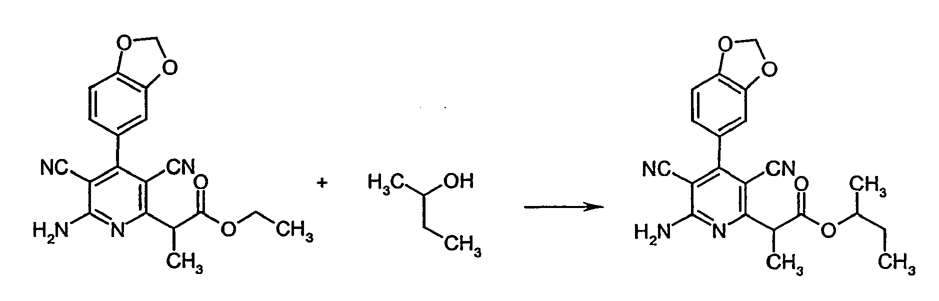

Ausbeute: 20 % d.Th.
ESI (positiv) ber. 392,41 gef. 392

### Beispiel 10

### 2-Amino-4-(1,3-benzodioxol-5-yl)-6-cyclopropyl-3,5-dicyano-pyridin

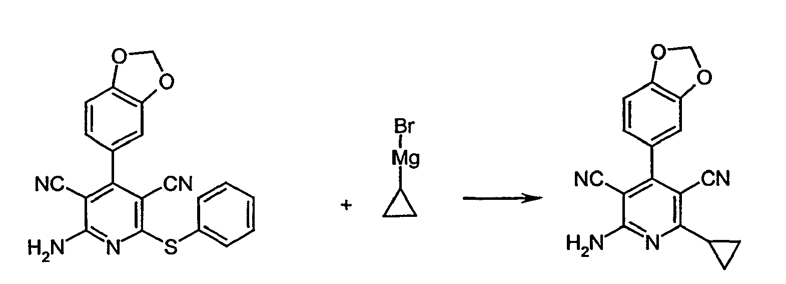

Unter Argonatmosphäre wird 2-Amino-4-(1,3-benzodioxol-5-yl)-6-(phenylsulfanyl)-3,5-dicyano-pyridin [Herstellung analog zu J.M. Quintela, J.L. Soto, Anales de Quimica 79, 368-372 (1983)] (100 mg, 0.27 mmol) in absolutem THF (3 ml) gelöst. Es wird 1,3-Bis(diphenylphosphino)-propan-dichlornickel(II) (4,4 mg, 0,008 mmol) zugegeben, wobei sich die Lösung rosa färbt. Beim langsamen Zutropfen des Cyclopropylmagnesiumbromids (1M Lösung in THF; 0.644 ml, 0,644 mmol) ist ein deutlicher Farbumschlag zu braunrot zu erkennen. Die Lösung wird für 3 Stunden auf 50°C erhitzt. Schon nach ca. 5 Minuten wird die Lösung grün. Es wird 1N Salzsäure (1 ml) zugegeben und anschließend mit Diethylether verdünnt. Festes Natriumcarbonat sowie Wasser werden zugegeben. Die Phasen werden getrennt und die organische Phase wird über Magnesiumsulfat getrocknet, filtriert und eingeengt. Die Mischung wird mittels Kieselgelsäulenchromatographie (Toluol:Ethylacetat = 2:1) getrennt.
Ausbeute: 15 mg (18 % d.Th.)
¹H-NMR (200 MHz, DMSO-d₆): δ =1.07 - 1.17 (m, 4H), 2.18 - 2.50 (m, 1H), 6.15 (s, 2H), 7.01 - 7.16 (m, 3H), 7.71 (bs, 2H).
ESI (positiv) ber. 304,31 gef. [M+H] 305,2

Die im Folgenden beschriebenen Beispielverbindungen 11 und 12 werden analog zu Beispiel 10 hergestellt:

### Beispiel 11

### 2-Amino-4-(1,3-benzodioxol-5-yl)-3,5-dicyano-6-(1-methyl-2-phenylethyl)-pyridin

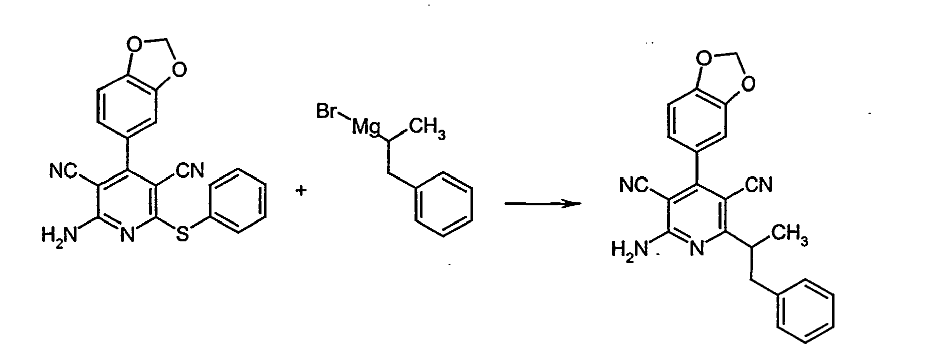

Ausbeute: 43 % d.Th.
ESI (positiv) ber. 382,421 gef. [M+H] 383

### Beispiel 12

### 2-Amino-4-(1,3-benzodioxol-5-yl)-6-(cyclopentyl)-3,5-dicyano-pyridin

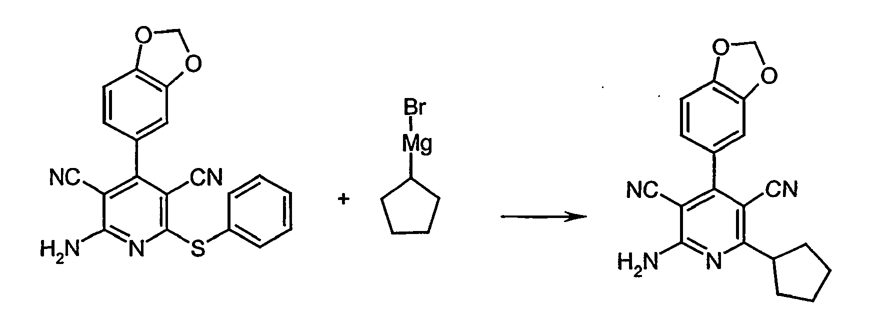

Ausbeute: 27 % d.Th.
ESI (positiv) ber. 332,361 gef. [M+H] 333,1

### Beispiel 13

### 2-[6-Amino-4-(1,3-benzodioxol-5-yl)-3,5-dicyano-2-pyridinyl]-propionsäure-cyclobutylester

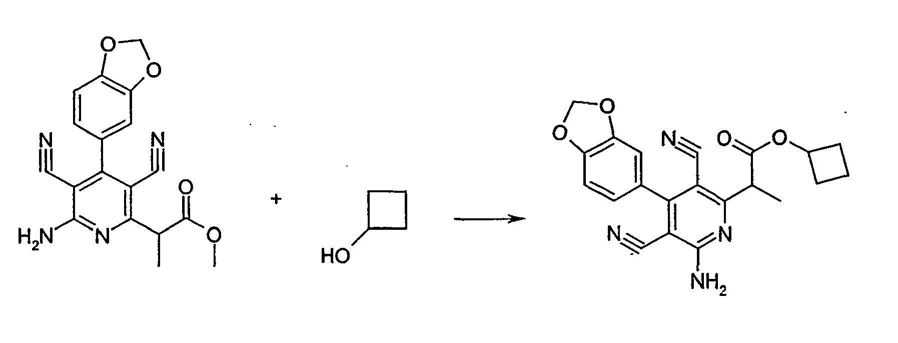

2-[6-Amino-4-(1,3-benzodioxol-5-yl)-3,5-dicyano-2-pyridinyl]-propionsäuremethyl-ester (aus Beispiel 6) (100 mg, 0,29 mmol) wird in Cyclobutanol (5 ml) mit einer katalytischen Menge Natriumhydrid für 2.5 Stunden unter Rückfluss erhitzt. Die Isolierung des Produkts erfolgt mittels RP-HPLC (Kromasilsäule 250 * 20 mm, C18, 10 µm; Acetonitril/Wasser-Gradient: 3 Minuten 10 %, dann innerhalb 30 Minuten auf 80 %, Flussrate: 25 ml*min⁻¹).
Ausbeute: 52 mg (46 % d.Th.).
¹H-NMR (200 MHz, DMSO-d₆): δ = 1.45 (d, 3H), 1.5 - 1.8 (m, 2H), 2.0 (m, 2H), 2.35 (m, 2H), 4.15 (q, 1H), 4.9 (q, 1H), 6.15 (s, 2H), 7.05 - 7.20 (m, 3H), 7.9 (bs, 2H).
ESI (positiv) ber. 390 gef. [M+H] 391

### Beispiel 14

### 2-Amino-4-(1,3-benzodioxol-5-yl)-6-(1-methyl-2-propenyl)-3,5-pyridindicarbo-nitril

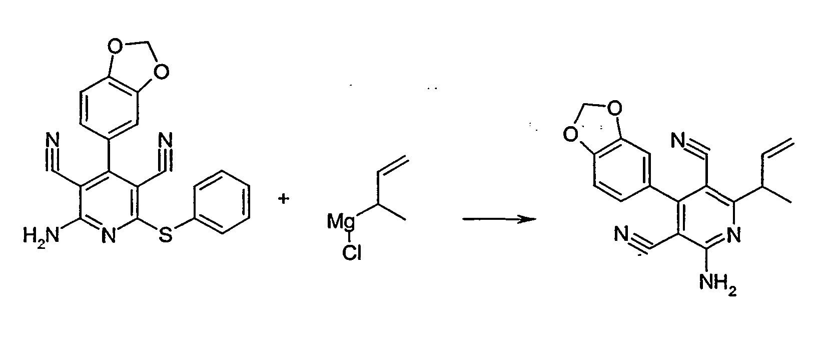

Herstellung analog zu Beispiel 10.
Ausbeute: 74.6 mg (29 % d.Th.).
¹H-NMR (200 MHz, DMSO-d₆): δ = 1.4 (d, 3H), 3.9 (q, 1H), 5.1 (m, 2H), 6.0 (m, 1H), 6.15 (s, 2H), 7.0 - 7.20 (m, 3H), 7.9 (bs, 2H).
ESI (positiv) ber. 318 gef. [M+H] 319

### Beispiel 15

### 2-Amino-4-(2,3-dihydro-1,4-benzodioxin-6-yl)-6-isopropyl-3,5-pyridindicarbo-nitril

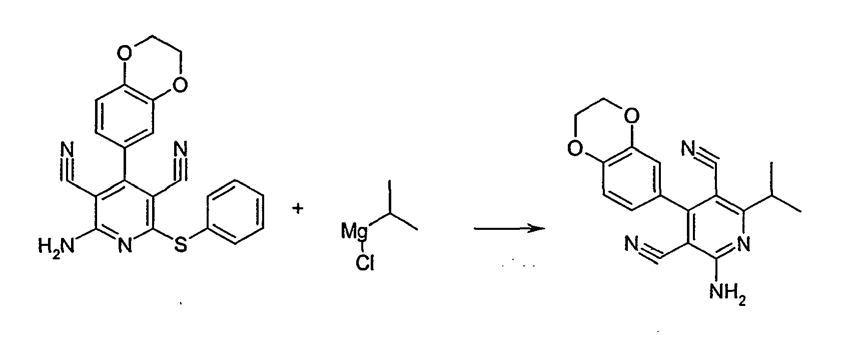

Herstellung analog zu Beispiel 10.
Ausbeute: 64 mg (25 % d.Th.).
¹H-NMR (200 MHz, DMSO-d₆): δ = 1.25 (d, 6H), 4.0 (q, 1H), 4.35 (s, 4H), 7.0 (m, 3H), 7.8 (bs, 2H).
ESI (positiv) ber. 320 gef. [M+H] 321

### Beispiel 4

### Stufe 1

### [6-Amino-4-(2,3-dihydro-1,4-benzodioxin-6-yl)-3,5-dicyano-2-pyridinyl]-essigsäureethylester

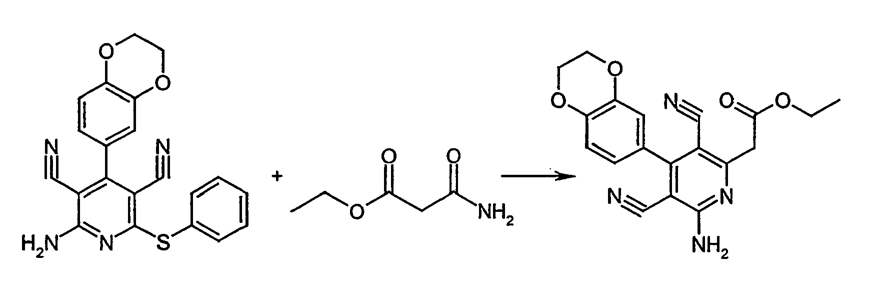

Herstellung analog zu Beispiel 1, Stufe 1.
Ausbeute: 462 mg (49 % d.Th.).
ESI (positiv) ber. 364 gef. [M+H] 365

### Stufe 2

### 2-[6-Amino-4-(2,3-dihydro-1,4-benzodioxin-6-yl)-3,5-dieyano-2-pyridinyl]-propionsäureethylester

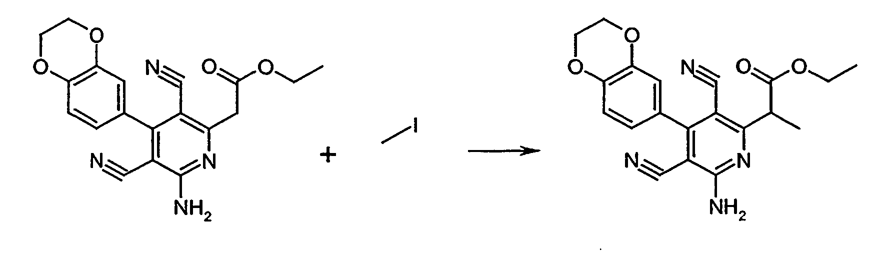

Herstellung analog zu Beispiel 1, Stufe 2.
Ausbeute: 258 mg (56 % d.Th.).
¹H-NMR (200 MHz, DMSO-d₆): δ = 1.15 (tr, 3H), 1.45 (d, 6H), 4.05 (q, 1H), 4.15 (q, 2H), 4.35 (s, 4H), 7.0 (m, 3H), 7.9 (bs, 2H).
ESI (positiv) ber. 378 gef. [M+H] 379

## Patentansprüche

1. Verbindungen der Formel (I) 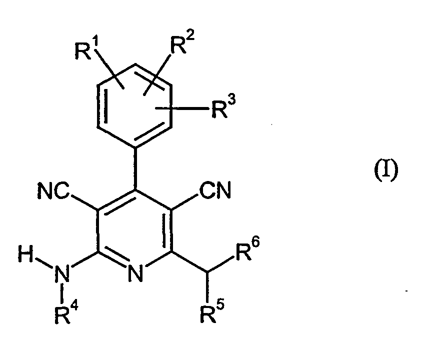 worin
R¹, R² und R³ unabhängig voneinander (C₁-C₈)-Alkyl, das bis zu dreifach durch Hydroxy, (C₁-C₄)-Alkoxy, (C₃-C₇)-Cycloalkyl, (C₂-C₄)-Alkenyl, (C₂-C₄)-Alkinyl, Halogen oder (C₆-C₁₀)-Aryloxy substituiert sein kann, (C₆-C₁₀)-Aryl, das bis zu dreifach durch Halogen, Nitro, (C₁-C₄)-Alkoxy, Carboxyl, (C₁-C₄)-Alkoxycarbonyl oder Mono- oder Di-(C₁-C₄)-alkylamino substituiert sein kann, (C₁-C₈)-Alkoxy, das durch Hydroxy, (C₁-C₄)-Alkoxy, (C₃-C₇)-Cycloalkyl, (C₂-C₄)-Alkenyl, (C₆-C₁₀)-Aryl, 5- oder 6-gliedriges Heteroaryl mit bis zu drei Heteroatomen aus der Reihe N, O und/oder S, (C₆-C₁₀)-Aryloxy, Halogen, Cyano, (C₁-C₄)-Alkoxycarbonyl, (C₁-C₄)-Alkanoyloxy, Amino oder Mono- oder Di-(C₁-C₄)-alkylamino substituiert sein kann, Wasserstoff, Hydroxy, Halogen, Nitro, Cyano oder -NH-C(O)- R⁷ bedeuten,
worin
R⁷ (C₁-C₈)-Alkyl, das durch Hydroxy oder (C₁-C₄)-Alkoxy substituiert sein kann, (C₃-C₇)-Cycloalkyl oder (C₆-C₁₀)-Aryl, das bis zu dreifach durch, unabhängig voneinander, durch Halogen, Nitro, (C₁-C₄)-Alkoxy, Carboxyl, (C₁-C₄)-Alkoxy-carbonyl oder Mono- oder Di-(C₁-C₄)-alkylamino substituiert sein kann, bedeutet,
oder
R¹ und R² an benachbarte Phenylringatome gebunden sind und mit den beiden Ringkohlenstoffatomen gemeinsam einen 5- bis 7-gliedrigen gesättigten oder partiell ungesättigten Heterocyclus mit einem oder zwei Heteroatomen aus der Reihe N, O und/oder S bilden, der durch (C₁-C₄)-Alkyl oder Oxo substituiert sein kann,
R⁴ Wasserstoff, (C₁-C₈)-Alkyl, das durch Hydroxy, (C₁-C₄)-Alkoxy, (C₃-C₇)-Cycloalkyl, (C₆-C₁₀)-Aryl, 5- oder 6-gliedriges gesättigtes oder partiell ungesättigtes Heterocyclyl mit bis zu drei Heteroatomen aus der Reihe N, O und/oder S oder 5- bis 10-gliedriges Heteroaryl mit bis zu drei Heteroatomen aus der Reihe N, O und/oder S substituiert sein kann, oder (C₃-C₇)-Cycloalkyl, das durch Hydroxy oder (C₁-C₈)-Alkyl substituiert sein kann, bedeutet,
R⁵ (C₁-C₄)-Alkyl oder (C₁-C₄)-Alkoxy, die ein- oder zweifach, unabhängig voneinander, durch Hydroxy, (C₁-C₄)-Alkoxy, (C₃-C₇)-Cycloalkyl, (C₆-C₁₀)-Aryl oder 5- bis 10-gliedriges Heteroaryl mit bis zu drei Heteroatomen aus der Reihe N, O und/oder S substituiert sein können, wobei Aryl und Heteroaryl ihrerseits durch Halogen, (C₁-C₄)-Alkyl, (C₁-C₄)-Alkoxy, Amino, Mono- oder Di-(C₁-C₄)-allcylamino, Nitro, Cyano, Trifluormethyl oder Hydroxy substituiert sein können, oder (C₂-C₄)-Alkenyl bedeutet,
R⁶ (C₁-C₈)-Alkyl, das durch Hydroxy, (C₁-C₄)-Alkoxy, (C₃-C₇)-Cyclo-alkyl, (C₂-C₄)-Alkenyl, -CO-O-R⁸, (C₆-C₁₀)-Aryl oder 5- bis 10-gliedriges Heteroaryl mit bis zu drei Heteroatomen aus der Reihe N, O und/oder S substituiert sein kann wobei Aryl und Heteroaryl ihrerseits durch Halogen, (C₁-C₄)-Alkyl, (C₁-C₄)-Alkoxy, Amino, Mono- oder Di-(C₁-C₄)-alkylamino, Nitro, Cyano, Trifluormethyl oder Hydroxy substituiert sein können, (C₃-C₇)-Cycloalkyl oder -CO-O-R⁸ bedeutet,
worin
R⁸ Wasserstoff, (C₁-C₈)-Alkyl, das durch Hydroxy oder (C₁-C₄)-Alkoxy substituiert sein kann, (C₃-C₇)-Cycloalkyl oder (C₆-C₁₀)-Aryl, das bis zu dreifach, unabhängig voneinander, durch Halogen, Nitro, (C₁-C₄)-Alkoxy, Carboxyl, (C₁-C₄)-Alkoxy-carbonyl oder Mono- oder Di-(C₁-C₄)-alkylamino substituiert sein kann, bedeutet,
oder
R⁵ und R⁶ gemeinsam mit dem Kohlenstoffatom, an das sie gebunden sind, einen 3- bis 7-gliedrigen, gesättigten oder partiell ungesättigten Ring bilden, der ein oder zwei Heteroatome aus der Reihe N, O und/oder S im Ring enthalten kann und der ein- bis dreifach, unabhängig voneinander, durch Oxo, Fluor, Chlor, Brom, Hydroxy, (C₁-C₆)-Alkyl oder (C₁-C₆)-Alkoxy substituiert sein kann,
und ihre Salze, Hydrate, Hydrate der Salze und Solvate.

2. Verbindungen nach Anspruch 1
worin
R¹ und R² unabhängig voneinander, Wasserstoff, (C₁-C₆)-Alkyl, (C₁-C₆)-Alkoxy, das durch Hydroxy, (C₁-C₄)-Alkoxy, (C₁-C₄)-Alkanoyloxy oder Cyclopropyl substituiert sein kann, Wasserstoff, Hydroxy, Fluor, Chlor, Nitro oder -NH-C(O)-CH₃ bedeuten
oder
R¹ und R² an benachbarte Phenylringatome gebunden sind und für eine Gruppe -O-CH₂-O- oder -O-CH₂-CH₂-O- stehen,
R³ Wasserstoff bedeutet,
R⁴ Wasserstoff, (C₁-C₄)-Alkyl, das durch Hydroxy, (C₁-C₄)-Alkoxy oder Cyclopropyl substituiert sein kann, oder Cyclopropyl bedeutet,
R⁵ (C₁-C₄)-Alkyl, das ein- oder zweifach, unabhängig voneinander, durch (C₃-C₆)-Cycloalkyl, Phenyl, das seinerseits durch Fluor, Trifluor-methyl oder (C₁-C₄)-Alkoxy substituiert sein kann, Pyridyl, Furyl oder Thienyl substituiert sein kann, oder (C₂-C₄)-Alkenyl bedeutet
und
R⁶ (C₁-C₄)-Alkyl oder (C₁-C₄)-Alkoxycarbonyl bedeutet
oder
R⁵ und R⁶ gemeinsam mit dem Kohlenstoffatom, an das sie gebunden sind, einen 3 bis 7 gliedrigen, gesättigten oder partiell ungesättigten Ring bilden, der ein Heteroatom aus der Reihe N, O oder S im Ring enthalten kann
und ihre Salze, Hydrate, Hydrate der Salze und Solvate.

3. Verbindungen nach Anspruch 1 oder 2
worin
R¹ Wasserstoff, Chlor, Nitro, Methyl, Methoxy, Ethoxy, n-Propoxy, Isopropoxy, n-Butoxy, wobei die Alkoxyreste ihrerseits durch Hydroxy, Methoxy, Ethoxy, n-Propoxy, Isopropoxy, n-Butoxy, -O-C(O)-CH₃ oder Cyclopropyl substituiert sein können, oder -NH-C(O)-CH₃ bedeutet,
R² Wasserstoff bedeutet
oder
R¹ und R² an benachbarte Phenylringatome gebunden sind und für eine Gruppe -O-CH₂-O- stehen,
R³ Wasserstoff bedeutet,
R⁴ Wasserstoff, Methyl, Ethyl, n-Propyl, Isopropyl, n-Butyl, wobei die Alkylreste ihrerseits durch Hydroxy, Methoxy, Ethoxy, n-Propoxy, Isopropoxy, n-Butoxy oder Cyclopropyl substituiert sein können, oder Cyclopropyl bedeutet,
R⁵ Methyl, Ethyl, n-Propyl, Isopropyl, n-Butyl, wobei die Alkylreste ihrerseits ein- oder zweifach, unabhängig voneinander, durch Cyclopropyl, Phenyl, das seinerseits durch Fluor, Trifluormethyl oder Methoxy substituiert sein kann, Pyridyl, Furyl oder Thienyl substituiert sein können, Ethenyl, Propenyl oder Butenyl bedeutet
und
R⁶ Methyl, Ethyl, n-Propyl, Isopropyl, n-Butyl, Methoxycarbonyl, Ethoxycarbonyl, n-Propoxycarbonyl, Isopropoxycarbonyl, n-Butoxy-carbonyl oder Isobutoxycarbonyl bedeutet
oder
R⁵ und R⁶ gemeinsam mit dem Kohlenstoffatom, an das sie gebunden sind, einen Cyclopropyl-, Cyclobutyl, Cyclopentyl- oder Cyclohexylring bilden
und ihre Salze, Hydrate, Hydrate der Salze und Solvate.

4. Verfahren zur Herstellung von Verbindungen der Formel (I), wie in Anspruch 1 definiert, **dadurch gekennzeichnet, dass** man entweder
[A] Verbindungen der Formel (II) 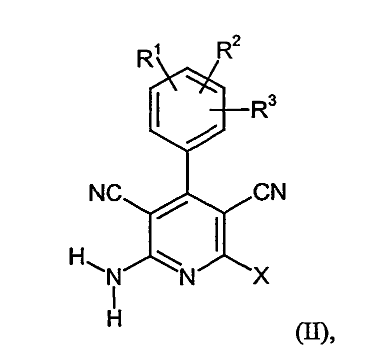 in welcher
R¹, R² und R³ die in Anspruch 1 angegebene Bedeutung haben und X für eine Abgangsgruppe steht,
zunächst mit Malonsäureamidethylester (III)
H₂N-C(O)- CH₂ - C(O)-O-C₂H₅ (III),
zu Verbindungen der Formel (IV) 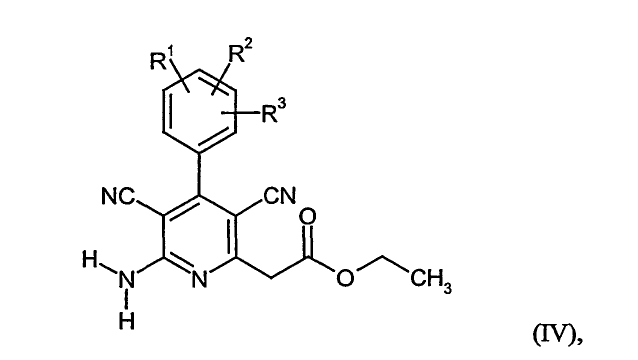 in welcher
R¹, R² und R³ die in Anspruch 1 angegebene Bedeutung haben,
umsetzt
und dann mit Verbindungen der Formel (V)
R⁵-Y (V);
in welcher
R⁵ die in Anspruch 1 angegebene Bedeutung hat und Y für eine Abgangsgruppe steht,
zu Verbindungen der Formel (I) umsetzt,
in welcher
R¹, R², R³, R⁴ und R⁵ die in Anspruch 1 angegebene Bedeutung haben und R⁶ für einen Rest -C(O)-O-C₂H₅ steht,
und gegebenenfalls anschließend mit Verbindungen der Formel (VI)
R⁸ - OH (VI),
in welchen R⁸ die in Anspruch 1 angegebene Bedeutung hat,
zu Verbindungen der Formel (I) umsetzt,
in welcher
R⁶ für einen Rest -C(O)-O-R⁸ steht und R¹, R², R³, R⁴, R⁵ und R⁸ die in Anspruch 1 angegebene Bedeutung haben,
oder
[B] Verbindungen der Formel (II)
mit Grignardverbindungen der Formel (VII) 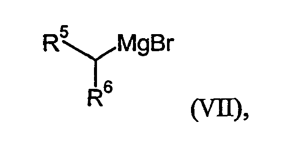
in welcher
R⁵ und R⁶ die in Anspruch 1 angegebene Bedeutung haben,
zu Verbindungen der Formel (I) umsetzt,
in welcher
R¹, R², R³, R⁵ und R⁶ die in Anspruch 1 angegebene Bedeutung haben und R⁴ für Wasserstoff steht,
und gegebenfalls anschließend mit Verbindungen der Formel (VIII)
R⁴ - Y' (VIII),
in welcher
R⁴ die in Anspruch 1 angegebene Bedeutung und Y' für eine Abgangsgruppe steht,
umsetzt.

5. Verbindungen der Formel (I), wie in Anspruch 1 definiert, zur Prophylaxe und/oder Behandlung von Erkrankungen.

6. Zusammensetzung, enthaltend mindestens eine Verbindungen der Formel (I), wie in Anspruch 1 definiert, und mindestens einen weiteren Hilfsstoff.

7. Verwendung von Verbindungen der Formel (I), wie in Anspruch 1 definiert, zur Herstellung von Arzneimitteln zur Prophylaxe und/oder Behandlung von Erkrankungen des Herzkreislaufsystems (kardiovaskulären Erkrankungen).

8. Verwendung von Verbindungen der Formel (I), wie in Anspruch 1 definiert, zur Herstellung von Arzneimitteln zur Prophylaxe und/oder Behandlung von Diabetes.

## Claims

1. Compounds of the formula (I) 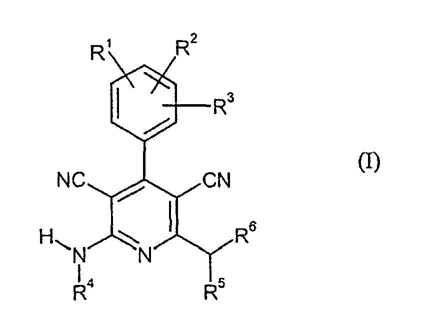 in which
R¹, R² and R³ independently of one another represent (C₁-C₈)-alkyl which may be substituted up to three times by hydroxyl, (C₁-C₄)-alkoxy, (C₃-C₇)-cycloalkyl, (C₂-C₄)-alkenyl, (C₂-C₄)-alkynyl, halogen or (C₆-C₁₀)-aryloxy, (C₆-C₁₀)-aryl which may be substituted up to three times by halogen, nitro, (C₁-C₄)-alkoxy, carboxyl, (C₁-C₄)-alkoxycarbonyl or mono- or di-(C₁-C₄)-alkylamino, (C₁-C₈)-alkoxy which may be substituted by hydroxyl, (C₁-C₄)-alkoxy, (C₃-C₇)-cycloalkyl, (C₂-C₄)-alkenyl, (C₆-C₁₀)-aryl, 5- or 6-membered heteroaryl having up to three heteroatoms from the group consisting of N, O and/or S, (C₆-C₁₀-aryloxy, halogen, cyano, (C₁-C₄)-alkoxycarbonyl, (C₁-C₄)-alkanoyloxy, amino or mono- or di-(C₁-C₄)-alkylamino, hydrogen, hydroxyl, halogen, nitro, cyano or -NH-C(O)-R⁷,
in which
R⁷ represents (C₁-C₈)-alkyl which may be substituted by hydroxyl or (C₁-C₄)-alkoxy, (C₃-C₇)-cycloalkyl or (C₆-C₁₀)-aryl which may be substituted up to three times, independently of one another, by halogen, nitro, (C₁-C₄)-alkoxy, carboxyl, (C₁-C₄)-alkoxycarbonyl or mono- or di-(C₁-C₄)-alkylamino,
or
R¹ and R² are attached to adjacent phenyl ring atoms and, together with the two ring carbon atoms, form a 5- to 7-membered saturated or partially unsaturated heterocycle having one or two heteroatoms from the group consisting of N, O and/or S which may be substituted by (C₁-C₄)-alkyl or oxo,
R⁴ represents hydrogen, (C₁-C₈)-alkyl which may be substituted by hydroxyl, (C₁-C₄)-alkoxy, (C₃-C₇) -cycloalkyl, (C₆-C₁₀)-aryl, 5- or 6-membered saturated or partially unsaturated heterocyclyl having up to three heteroatoms from the group consisting of N, O and/or S or 5- to 10-membered heteroaryl having up to three heteroatoms from the group consisting of N, O and/or S, or (C₃-C₇)-cycloalkyl which may be substituted by hydroxyl or (C₁-C₈)-alkyl,
R⁵ represents (C₁-C₄)-alkyl or (C₁-C₄)-alkoxy which may be mono- or disubstituted, independently of one another, by hydroxyl, (C₁-C₄)-alkoxy, (C₃-C₇)-cycloalkyl, (C₆-C₁₀)-aryl or 5- to 10-membered heteroaryl having up to three heteroatoms from the group consisting of N, O and/or S, where aryl and heteroaryl for their part may be substituted by halogen, (C₁-C₄)-alkyl, (C₁-C₄)-alkoxy, amino, mono- or di-(C₁-C₄)-alkylamino, nitro, cyano, trifluoromethyl or hydroxyl, or (C₂-C₄)-alkenyl,
R⁶ represents (C₁-C₈)-alkyl which may be substituted by hydroxyl, (C₁-C₄)-alkoxy, (C₃-C₇)-cycloalkyl, (C₂-C₄)-alkenyl, -CO-O-R⁸, (C₆-C₁₀)-aryl or 5- to 10-membered heteroaryl having up to three heteroatoms from the group consisting of N, O and/or S, where aryl and heteroaryl for their part may be substituted by halogen, (C₁-C₄)-alkyl, (C₁-C₄)-alkoxy, amino, mono- or di-(C₁-C₄)-alkylamino, nitro, cyano, trifluoromethyl or hydroxyl, (C₃-C₇)-cycloalkyl or -CO-O-R⁸,
in which
R⁸ represents hydrogen, (C₁-C₈)-alkyl which may be substituted by hydroxyl or (C₁-C₄)-alkoxy, (C₃-C₇)-cycloalkyl or (C₆-C₁₀)-aryl which may be substituted up to three times, independently of one another, by halogen, nitro, (C₁-C₄)-alkoxy, carboxyl, (C₁-C₄)-alkoxycarbonyl or mono- or di-(C₁-C₄)-alkylamino,
or
R⁵ and R⁶ together with the carbon atom to which they are attached form a 3- to 7-membered saturated or partially unsaturated ring which may contain one or two heteroatoms from the group consisting of N, O and/or S in the ring and which may be mono- to trisubstituted, independently of one another, by oxo, fluorine, chlorine, bromine, hydroxyl, (C₁-C₆)-alkyl or (C₁-C₆)-alkoxy,
and their salts, hydrates, hydrates of the salts and solvates.

2. Compounds according to claim 1
in which
R¹ and R² independently of one another represent hydrogen, (C₁-C₆)-alkyl, (C₁-C₆)-alkoxy which may be substituted by hydroxyl, (C₁-C₄)-alkoxy, (C₁-C₄)-alkanoyloxy or cyclopropyl, hydrogen, hydroxyl, fluorine, chlorine, nitro or -NH-C(O)-CH₃
or
R¹ and R² are attached to adjacent phenyl ring atoms and represent a group -O-CH₂-O- or -0-CH₂-CH₂-O-,
R³ represents hydrogen,
R⁴ represents hydrogen, (C₁-C₄)-alkyl which may be substituted by hydroxyl, (C₁-C₄)-alkoxy or cyclopropyl, or cyclopropyl,
R⁵ represents (C₁-C₄)-alkyl which may be mono- or disubstituted, independently of one another, by (C₃-C₆)-cycloalkyl, phenyl, which for its part may be substituted by fluorine, trifluoromethyl or (C₁-C₄)-alkoxy, pyridyl, furyl or thienyl, or (C₂-C₄)-alkenyl,
and
R⁶ represents (C₁-C₄)-alkyl or (C₁-C₄)-alkoxycarbonyl
or
R⁵ and R⁶ together with the carbon atom to which they are attached form a 3- to 7-membered saturated or partially unsaturated ring which may contain a heteroatom from the group consisting of N, O or S in the ring
and their salts, hydrates, hydrates of the salts and solvates.

3. Compounds according to claim 1 or 2
in which
R¹ represents hydrogen, chlorine, nitro, methyl, methoxy, ethoxy, n-propoxy, isopropoxy, n-butoxy, where the alkoxy radicals for their part may be substituted by hydroxyl, methoxy, ethoxy, n-propoxy, isopropoxy, n-butoxy, -0-C(O)-CH₃ or cyclopropyl, or -NH-C(O)-CH₃,
R² represents hydrogen
or
R¹ and R² are attached to adjacent phenyl ring atoms and represent a group -O-CH₂-O-,
R³ represents hydrogen,
R⁴ represents hydrogen, methyl, ethyl, n-propyl, isopropyl, n-butyl, where the alkyl radicals for their part may be substituted by hydroxyl, methoxy, ethoxy, n-propoxy, isopropoxy, n-butoxy or cyclopropyl, or cyclopropyl,
R⁵ represents methyl, ethyl, n-propyl, isopropyl, n-butyl, where the alkyl radicals for their part may be mono- or disubstituted, independently of one another, by cyclopropyl, phenyl, which for its part may be substituted by fluorine, trifluoromethyl or methoxy, pyridyl, furyl or thienyl, ethenyl, propenyl or butenyl
and
R⁶ represents methyl, ethyl, n-propyl, isopropyl, n-butyl, methoxycarbonyl, ethoxycarbonyl, n-propoxycarbonyl, isopropoxycarbonyl, n-butoxycarbonyl or isobutoxycarbonyl
or
R⁵ and R⁶ together with the carbon atom to which they are attached form a cyclopropyl, cyclobutyl, cyclopentyl or cyclohexyl ring and their salts, hydrates, hydrates of the salts and solvates.

4. Process for preparing compounds of the formula (I) as defined in claim 1, **characterized in that** either
[A] compounds of the formula (II) 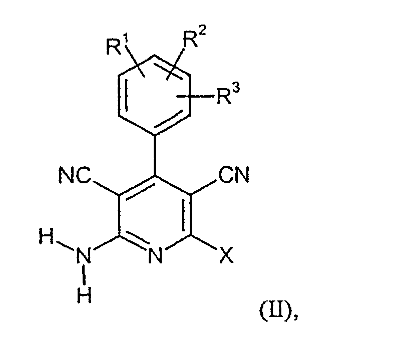 in which
R¹, R² and R³ are as defined in claim 1 and X represents a leaving group,
are initially reacted with ethyl malonamide (III)
H₂N-C(O)-CH₂-C(O)-O-C₂H₅ (III)
to give compounds of the formula (IV) 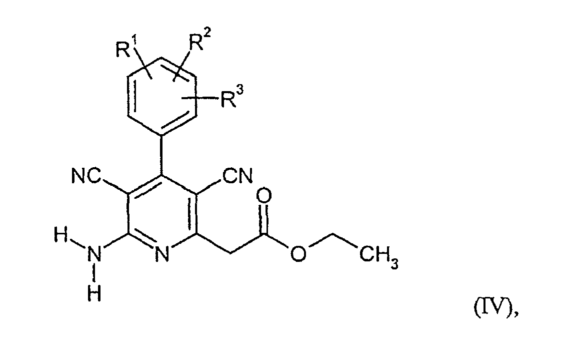 in which
R¹, R² and R³ are as defined in claim 1,
and then with compounds of the formula (V)
R⁵ - Y (V)
in which
R⁵ is as defined in claim 1 and Y represents a leaving group
to give compounds of the formula (I)
in which
R¹, R², R³, R⁴ and R⁵ are as defined in claim 1 and R⁶ represents a radical -C(O)-O-C₂H₅,
and, if appropriate, subsequently with compounds of the formula (VI)
R⁸ - OH (VI)
in which R⁸ is as defined in claim 1
to give compounds of the formula (I)
in which
R⁶ represents a radical -C(O)-O-R⁸ and R¹, R², R³, R⁴, R⁵ and R⁸ are as defined in claim 1,
or
[B] compounds of the formula (II)
are reacted with Grignard compounds of the formula (VII) 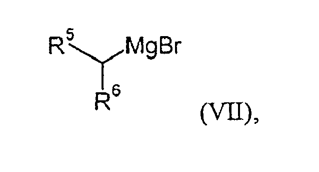
in which
R⁵ and R⁶ are as defined in claim 1
to give compounds of the formula (I)
in which
R¹, R², R³, R⁵ and R⁶ are as defined in claim 1 and R⁴ represents hydrogen,
and, if appropriate, subsequently with compounds of the formula (VIII)
R⁴ - Y' (VIII)
in which
R⁴ is as defined in claim 1 and Y' represents a leaving group.

5. Compounds of the formula (I) as defined in claim 1 for the prophylaxis and/or treatment of disorders.

6. Composition, comprising at least one compound of the formula (I) as defined in claim 1 and at least one auxiliary.

7. Use of compounds of the formula (I) as defined in claim 1 for preparing medicaments for the prophylaxis and/or treatment of disorders of the cardiovascular system (cardiovascular disorders).

8. Use of compounds of the formula (I) as defined in claim 1 for preparing medicaments for the prophylaxis and/or treatment of diabetes.

## Revendications

1. Composés de formule (I) 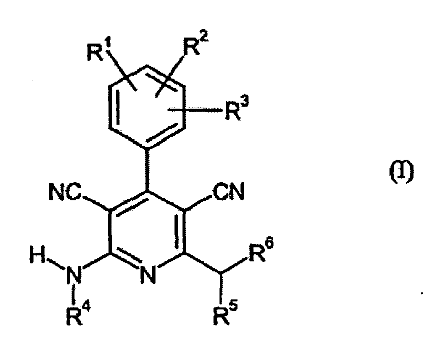 dans laquelle
R¹, R² et R³ représentent, indépendamment les uns des autres, un reste alkyle en C₁ à C₈, qui peut être substitué jusqu'à trois fois par un radical hydroxy, alkoxy en C₁ à C₄, cycloalkyle en C₃ à C₇, alcényle en C₂ à C₄, alcynyle en C₂ à C₄, halogéno ou aryloxy en C₆ à C₁₀, un reste aryle en C₆ à C₁₀, qui peut être substitué jusqu'à trois fois par un radical halogéno, nitro, alkoxy en C₁ à C₄, carboxyle, (alkoxy en C₁ à C₄)carbonyle ou mono- ou di- (alkyle en C₁ à C₄)amino, un reste alkoxy en C₁ à C₈, qui peut être substitué par un radical hydroxy, alkoxy en C₁ à C₄, cycloalkyle en C₃ à C₇, alcényle en C₂ à C₄, aryle en C₆ à C₁₀, hétéroaryle pentagonal ou hexagonal comprenant jusqu'à trois hétéroatomes de la série N, O et/ou S, aryloxy en C₆ à C₁₀, halogéno, cyano, (alkoxy en C₁ à C₄)carbonyle, alcanoyloxy en C₁ à C₄, amino ou mono-ou di-(alkyle en C₁ à C₄)amino, un reste hydrogène, hydroxy, halogéno, nitro, cyano ou un groupe -NH-C(O)-R⁷,
dans lequel
R⁷ représente un reste alkyle en C₁ à C₈, qui peut être substitué par un radical hydroxy ou alkoxy en C₁ à C₄, un reste cycloalkyle en C₃ à C₇ ou aryle en C₆ à C₁₀, qui peut être substitué jusqu'à trois fois indépendamment les unes des autres par un radical halogéno, nitro, alkoxy en C₁ à C₄, carboxyle, (alkoxy en C₁ à C₄)carbonyle ou mono- ou di-(alkyle en C₁ à C₄)amino,
ou bien
R¹ et R² sont liés à des atomes contigus du noyau phényle et forment conjointement avec les deux atomes de carbone du noyau un hétérocycle pentagonal à heptagonal saturé ou partiellement insaturé, comprenant un ou deux hétéroatomes de la série N, O et/ou S, qui peut être substitué par un radical alkyle en C₁ à C₄ ou oxo,
R⁴ représente l'hydrogène, un reste alkyle en C₁ à C₈, qui peut être substitué par un radical hydroxy, alkoxy en C₁ à C₄, cycloalkyle en C₃ à C₇, aryle en C₆ à C₁₀, hétérocyclyle pentagonal ou hexagonal saturé ou partiellement insaturé comprenant jusqu'à trois hétéroatomes de la série N, O et/ou S ou un radical hétéroaryle de 5 à 10 chaînons comprenant jusqu'à trois hétéroatomes de la série N, O et/ou S, ou un reste cycloalkyle C₃ à C₇ qui peut être substitué par un radical hydroxy ou alkyle en C₁ à C₈,
R⁵ représente un reste alkyle en C₁ à C₄ ou un reste alkoxy en C₁ à C₄, qui peuvent être substitués une ou deux fois, indépendamment l'un de l'autre, par un radical hydroxy, alkoxy en C₁ à C₄, cycloalkyle en C₃ à C₇, aryle en C₆ à C₁₀ ou hétéroaryle de 5 à 10 chaînons comprenant jusqu'à trois hétéroatomes de la série N, O et/ou S, les radicaux aryle et hétéroaryle pouvant eux-mêmes être substitués par un radical halogéno, alkyle en C₁ à C₄, alkoxy en C₁ à C₄, amino, mono- ou di-(alkyle en C₁ à C₄)amino, nitro, cyano, trifluorométhyle ou hydroxy, ou un reste alcényle en C₂ à C₄,
R⁶ représente un reste alkyle en C₁ à C₈, qui peut être substitué par un radical hydroxy, alkoxy en C₁ à C₄, cycloalkyle en C₃ à C₇, alcényle en C₂ à C₄, -CO-O-R⁸, aryle en C₆ à C₁₀ ou hétéroaryle de 5 à 10 chaînons comprenant jusqu'à trois hétéroatomes de la série N, O et/ou S, les radicaux aryle et hétéroaryle pouvant eux-mêmes être substitués par un radical halogéno, alkyle en C₁ à C₄, alkoxy en C₁ à C₄, amino, mono- ou di-(alkyle en C₁ à C₄)amino, nitro, cyano, trifluorométhyle ou hydroxy, un reste cycloalkyle en C₃ à C₇ ou un reste -CO-O-R⁸,
dans lequel
R⁸ représente l'hydrogène, un reste alkyle en C₁ à C₈, qui peut être substitué par un radical hydroxy ou alkoxy en C₁ à C₄, un reste cycloalkyle en C₃ à C₇ ou aryle en C₆ à C₁₀, qui peut être substitué jusqu'à trois fois indépendamment les unes des autres par un radical halogéno, nitro, alkoxy en C₁ à C₄, carboxyle, (alkoxy en C₁ à C₄)carbonyle ou mono- ou di-(alkyle en C₁ à C₄)amino,
ou bien
R⁵ et R⁶ forment, conjointement avec l'atome de carbone auquel ils sont liés, un noyau trigonal à heptagonal saturé ou partiellement insaturé qui peut contenir dans le noyau un ou deux hétéroatomes de la série N, O et/ou S et qui peut être substitué une à trois fois, indépendamment l'une de l'autre, par un radical oxo, fluoro, chloro, bromo, hydroxy, alkyle en C₁ à C₆ ou alkoxy en C₁ à C₆,
et leurs sels, leurs hydrates, les hydrates de leurs sels et leurs produits de solvatation.

2. Composés suivant la revendication 1,
dans lesquels
R¹ et R² représentent, indépendamment l'un de l'autre, l'hydrogène, un reste alkyle en C₁ à C₆ ou alkoxy en C₁ à C₆, qui peut être substitué par un radical hydroxy, alkoxy en C₁ à C₄, alcanoyloxy en C₁ à C₄ ou cyclopropyle, un reste hydrogène, hydroxy, fluoro, chloro, nitro ou un groupe -NH-C(O)-CH₃
ou bien
R¹ et R² sont liés à des atomes contigus du noyau phényle et représentent un groupe -O-CH₂-O- ou -O-CH₂-CH₂-O-,
R³ représente l'hydrogène,
R⁴ représente l'hydrogène, un reste alkyle en C₁ à C₄, qui peut être substitué par un radical alkoxy en C₁ à C₄ ou cyclopropyle, ou un reste cyclopropyle,
R⁵ représente un reste alkyle en C₁ à C₄ qui peut être substitué une ou deux fois, indépendamment l'une de l'autre, par un radical cycloalkyle en C₃ à C₆ un reste phényle, qui peut lui-même être substitué par un radical fluoro, trifluorométhyle ou alkoxy en C₁ à C₄, un radical pyridyle, furyle ou thiényle, ou un reste alcényle en C₂ à C₄,
et
R⁶ représente un reste alkyle en C₁ à C₄ ou (alkoxy en C₁ à C₄) carbonyle
ou bien
R⁵ et R⁶ forment, conjointement avec l'atome de carbone auquel ils sont liés, un noyau trigonal à heptagonal saturé ou partiellement insaturé qui peut contenir un hétéroatome de la série N, O et/ou S dans le noyau,
et leurs sels, leurs hydrates, les hydrates de leurs sels et leurs produits de solvatation.

3. Composés suivant la revendication 1 ou 2,
dans lesquels,
R¹ représente l'hydrogène, le chlore, un reste nitro, méthyle, méthoxy, éthoxy, n-propoxy, isopropoxy, n-butoxy, les restes alkoxy pouvant eux-mêmes être substitués par un radical hydroxy, méthoxy, éthoxy, n-propoxy, isopropoxy, n-butoxy, -O-C(O)-CH₃ ou cyclopropyle, ou un groupe -NH-C(O)-CH₃
R² représente l'hydrogène,
ou bien
R¹ et R² sont liés à des atomes contigus du noyau phényle et représentent un groupe -O-CH₂-O-
R³ représente l'hydrogène,
R⁴ représente l'hydrogène, un reste méthyle, éthyle, n-propyle, isopropyle, n-butyle, les restes alkyle pouvant eux-mêmes être substitués par un radical hydroxy, méthoxy, éthoxy, n-propoxy, isopropoxy, n-butoxy ou cyclopropyle, ou un reste cyclopropyle,
R⁵ représente un reste méthyle, éthyle, n-propyle, isopropyle, n-butyle, les restes alkyle pouvant eux-mêmes être substitués une ou deux fois, indépendamment les uns des autres, par un radical cyclopropyle, phényle qui peut lui-même être substitué par un radical fluoro, trifluorométhyle ou méthoxy, un radical pyridyle, furyle ou thiényle, un reste éthényle, propényle ou butényle,
et
R⁶ représente un reste méthyle, éthyle, n-propyle, isopropyle, n-butyle, méthoxycarbonyle, éthoxycarbonyle, n-propoxycarbonyle, isopropoxycarbonyle, n-butoxycarbonyle ou isobutoxycarbonyle,
ou bien
R⁵ et R⁶ forment, conjointement avec l'atome de carbone auquel ils sont liés, un noyau cyclopropyle, cyclobutyle, cyclopentyle ou cyclohexyle,
et leurs sels, leurs hydrates, les hydrates de leurs sels et leurs produits de solvatation.

4. Procédé de production de composés de formule (I) telle que définie dans la revendication 1, **caractérisé en ce que**
[A] on fait tout d'abord réagir des composés de formule (II) 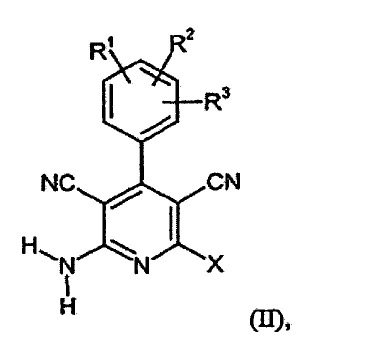 dans laquelle
R¹, R² et R³ ont la définition indiquée dans la revendication 1 et X représente un groupe partant, avec l'ester éthylique d'acide malonique (III)
H₂N-C(O)-CH₂-C(O)-O-C₂H₅ (III)
pour obtenir des composés de formule (IV) 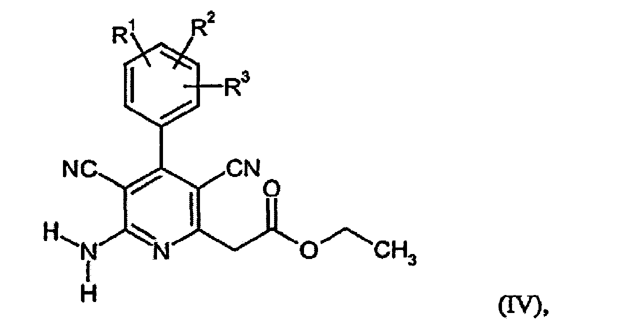 dans laquelle
R¹, R² et R³ ont la définition indiquée dans la revendication 1,
et ensuite avec des composés de formule (V)
R⁵-Y (V),
dans laquelle R⁵ a la définition indiquée dans la revendication 1 et Y est un groupe partant,
pour obtenir des composés de formule (I)
dans laquelle
R¹, R², R³, R⁴ et R⁵ ont la définition indiquée dans la revendication 1 et R⁶ représente un reste -C(O)-O-C₂H₅, qu'on fait ensuite réagir le cas échéant avec des composés de formule (VI)
R⁸-OH (VI)
dans laquelle R⁸ a la définition indiquée dans la revendication 1 pour obtenir des composés de formule (I)
dans laquelle
R⁶ représente un reste -C(O)-O-R⁸ et R¹, R², R³, R⁴, R⁵ et R⁸ ont la définition indiquée dans la revendication 1
ou bien
[B] on fait réagir des composés de formule (II) avec des composés de Grignard de formule (VII) 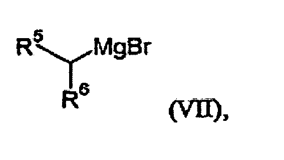 dans laquelle
R⁵ et R⁶ ont la définition indiquée dans la revendication 1,
pour obtenir des composés de formule (I)
dans laquelle
R¹, R², R³, R⁵ et R⁶ ont la définition indiquée dans la revendication 1 et R⁴ représente l'hydrogène, qu'on fait ensuite réagir éventuellement avec des composés de formule (VIII)
R⁴-Y' (VIII)
dans laquelle
R⁴ a la définition indiquée dans la revendication 1 et
Y' représente un groupe partant.

5. Composés de formule (I), telle que définie dans la revendication 1, destinés à la prophylaxie et/ou au traitement de maladies.

6. Composition contenant au moins un composé de formule (I) telle que définie dans la revendication 1, et au moins une autre substance active.

7. Utilisation de composés de formule (I), telle que définie dans la revendication 1, pour la préparation de médicaments destinés à la prophylaxie et/ou au traitement de maladies du système cardio-vasculaire (maladies cardio-vasculaires)

8. Utilisation de composés de formule (I), telle que définie dans la revendication 1, pour la préparation de médicaments destinés à la prophylaxie et/ou au traitement du diabète.
